(19) Europäisches Patentamt
European Patent Office
Office européen des brevets

(11) EP 3 070 093 B1

(12) # EUROPÄISCHE PATENTSCHRIFT

(45) Veröffentlichungstag und Bekanntmachung des Hinweises auf die Patenterteilung:
**07.02.2018 Patentblatt 2018/06**

(51) Int Cl.:
***C07F 9/09*** (2006.01)

(21) Anmeldenummer: **16164797.9**

(22) Anmeldetag: **24.10.2012**

(54) **NEUE ORGANOPHOSPHORVERBINDUNGEN AUF BASIS VON ANTHRACENTRIOL**
NOVEL ORGANOPHOSPHORUS COMPOUNDS BASED ON ANTHRACENTRIOL
NOUVELLES LIAISONS DE PHOSPHORE ORGANIQUE A BASE D'ANTHRACENTRIOLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.11.2011 DE 102011085883**

(43) Veröffentlichungstag der Anmeldung:
**21.09.2016 Patentblatt 2016/38**

(62) Dokumentnummer(n) der früheren Anmeldung(en) nach Art. 76 EPÜ:
**12778338.9 / 2 748 174**

(73) Patentinhaber: **Evonik Degussa GmbH**
**45128 Essen (DE)**

(72) Erfinder:
- **Christiansen, Andrea**
  **18119 Rostock (DE)**
- **Franke, Robert**
  **45772 Marl (DE)**
- **Fridag, Dirk**
  **45721 Haltern am See (DE)**
- **Hess, Dieter**
  **45770 Marl (DE)**
- **Kreidler, Burkard**
  **45219 Essen (DE)**
- **Selent, Detlef**
  **18059 Rostock (DE)**
- **Börner, Armin**
  **18059 Rostock (DE)**

(56) Entgegenhaltungen:
**WO-A1-95/30680 FR-A1- 2 215 221**
**US-A- 4 694 109**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 070 093 B1

## Beschreibung

Die vorliegende Erfindung betrifft Bis- und Trisphosphite enthalten mindestens ein Strukturelement auf Basis von Anthracentriol sowie deren Metallkomplexe, die Herstellung, sowie die Verwendung der Bis- und Trisphosphite als multidentate Verbindungen in katalytischen Reaktionen.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet, insbesondere Rhodium und Kobaltverbindungen. Die Hydroformylierung mit Rhodiumverbindungen bietet im Vergleich zur Katalyse mit Kobalt in der Regel den Vorteil höherer Selektivität und führt zu Produkten mit einer höheren Wertschöpfung. Bei der durch Rhodium katalysierten Hydroformylierung werden zumeist Zusammensetzungen eingesetzt, die aus Rhodium und bevorzugt aus trivalenten Phosphorverbindungen als Liganden bestehen. Bekannte Liganden sind beispielsweise Verbindungen aus den Klassen der Phosphine, Phosphite und Phosphonite mit jeweils dreiwertigen Phosphor $P^{III}$. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996.

Jede katalytisch aktive Zusammensetzung - auf Basis von Kobalt oder Rhodium - hat seine spezifischen Vorzüge. Je nach Einsatzstoff und Zielprodukt kommen daher unterschiedliche katalytisch aktive Zusammensetzungen zum Einsatz, wie folgende Beispiele zeigen. Arbeitet man mit Rhodium und Triphenylphosphin, lassen sich $\alpha$-Olefine bei niedrigeren Drücken hydroformylieren. Als Phosphor-haltiger Ligand wird in der Regel Triphenylphosphin im Überschuss verwendet, wobei ein hohes Ligand/Rhodium-Verhältnis erforderlich ist, um die Selektivität der Reaktion zum kommerziell erwünschten n-Aldehydprodukt zu erhöhen.

Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten erreicht. In WO 95/30680 werden zweizähnige Phosphinliganden und ihr Einsatz in der Katalyse, unter anderem auch in Hydroformylierungsreaktionen, offen gelegt. Ferrocenverbrückte Bisphosphine werden beispielsweise in den Patentschriften US 4 169 861, US 4 201 714 und US 4 193 943 als Liganden für Hydroformylierungen beschrieben.

Der Nachteil von zwei- und mehrzähnigen Phosphinliganden ist ein relativ hoher Aufwand, der zu ihrer Darstellung notwendig ist. Daher ist es oftmals nicht rentabel, solche Systeme in technischen Prozessen einzusetzen. Hinzu kommt eine vergleichsweise geringe Aktivität, die durch hohe Verweilzeiten reaktionstechnisch kompensiert werden muss. Dies wiederum führt zu unerwünschten Nebenreaktionen der Produkte.

Rhodium-Monophosphit-Komplexe in katalytisch aktiven Zusammensetzungen sind geeignet für die Hydroformylierung von verzweigten Olefinen mit innenständigen Doppelbindungen, jedoch ist die Selektivität für endständig oxierte Verbindungen gering. Aus EP 0 155 508 ist die Verwendung von bisarylensubstituierten Monophosphiten bei der rhodiumkatalysierten Hydroformylierung von sterisch gehinderten Olefinen, z. B. Isobuten, bekannt.

Katalytisch aktive Zusammensetzungen auf Basis von Rhodium-Bisphosphit-Komplexen sind geeignet für die Hydroformylierung von linearen Olefinen mit end- und innenständigen Doppelbindungen, wobei überwiegend endständig hydroformylierte Produkte entstehen. Dagegen werden verzweigte Olefine mit innenständigen Doppelbindungen nur im geringen Masse umgesetzt. Diese Phosphite ergeben bei ihrer Koordination an ein Übergangsmetallzentrum Katalysatoren von gesteigerter Aktivität, doch ist das Standzeitverhalten dieser katalytisch aktiven Zusammensetzungen, unter anderem wegen der Hydrolyseempfindlichkeit der Phosphitliganden, unbefriedigend. Durch den Einsatz von substituierten Bisaryldiolen als Bausteine für die Phosphitliganden, wie in EP 0 214 622 oder EP 0 472 071 beschrieben, konnten erhebliche Verbesserungen erreicht werden.

Der Literatur zufolge sind die katalytisch aktiven Zusammensetzungen dieser Liganden auf Basis von Rhodium äußerst aktiv in der Hydroformylierung von $\alpha$-Olefinen. In den Patenten US 4 668 651, US 4 748 261 und US 4 885 401 werden Polyphosphitliganden beschrieben, mit denen $\alpha$-Olefine, aber auch 2-Buten mit hoher Selektivität zu den terminal oxierten Produkten umgesetzt werden können. Zweizähnige Liganden dieses Typs wurden auch zur Hydroformylierung von Butadien eingesetzt (US 5 312 996).

Die in EP 1 294 731 offenbarten Bisphosphite weisen bei der Hydroformylierung von Octengemischen Olefinumsätze bis zu 98 % auf. Jedoch ist die ebenfalls gewünschte n-Selektivität zum Nonanal mit 36,8 % bis maximal 57,6 % verbesserungswürdig. Dies gilt umso mehr, als dass die Verwendung der katalytisch aktiven Zusammensetzung in technischen Prozessen eine Standzeit verlangt, welche sich in Tagen anstelle von Stunden bemisst.

Obgleich die genannten Bisphosphite gute Liganden für Rhodium-Hydroformylierungskatalysatoren sind, ist es wünschenswert, neue Liganden zu entwickeln.

Diese neuen Liganden sollen:

- hohe n-Selektivitäten bei der ‚Hydroformylierung von Olefinen oder olefinhaltigen Gemischen mit innenständigen

Doppelbindungen - somit isomerisierende Eigenschaften - aufweisen;

- zudem eine verbesserte Beständigkeit gegenüber inherenten Katalysatorgiften, wie z.B. Wasser, besitzen und somit eine verlängerte Standzeit bei der Verwendung in einer katalytisch aktiven Zusammensetzung zur Hydroformylierung ermöglichen;
- als auch die bekannte Neigung von Rhodium in katalytisch aktiven Zusammensetzungen zur Cluster-Bildung unterdrücken, womit ebenfalls eine verlängerte Standzeit bei der Verwendung in einer katalytisch aktiven Zusammensetzung zur Hydroformylierung realisiert wird.

Die Aufgabe wird gelöst durch eine erfindungsgemäße Verbindung, welche das Strukturelement (IV) aufweist:

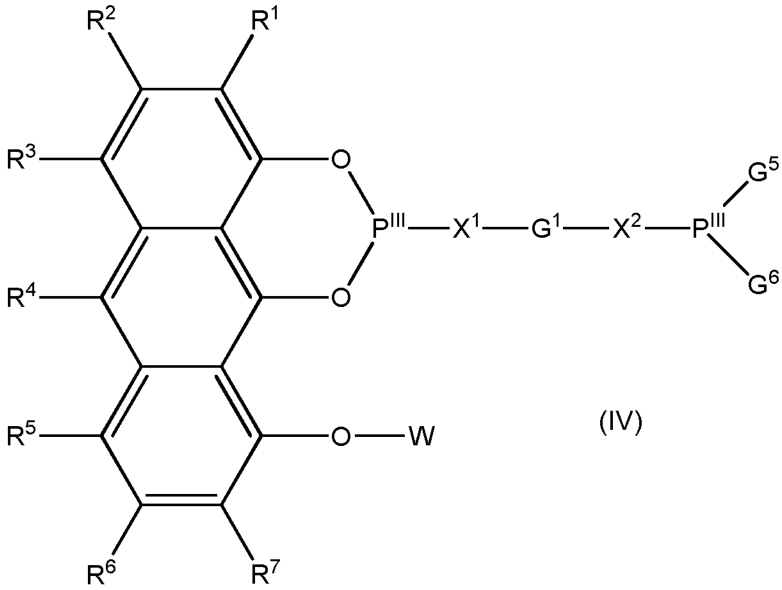

(IV)

mit $G^1$ = lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution;

wobei $X^1$, $X^2$ = O ist;

wobei $R^1$, $R^2$, $R^3$, $R^4$, $R^5$, $R^6$, $R^7$ ausgewählt sind aus: Wasserstoff, unsubstituierte oder substituierte, lineare oder verzweigte, aliphatische oder aromatische Kohlenwasserstoffgruppe; F, Cl, Br, I, $-OR^8$, $-C(O)R^9$, $-CO_2R^{10}$, $-CO_2M^1$, $-SR^{11}$,$-SOR^{12}$, $-SO_2R^{13}$, $-SO_3R^{14}$, $-SO_3M^2$, $-NR^{15}R^{16}$;

wobei $R^8$, $R^9$, $R^{10}$, $R^{11}$, $R^{12}$, $R^{13}$, $R^{14}$, $R^{15}$, $R^{16}$ ausgewählt sind aus: Wasserstoff, unsubstituierte oder substituierte, lineare oder verzweigte, aliphatische oder aromatische oder heteroaromatische oder kondensierte aromatische oder kondensierte aromatisch-heteroaromatische Kohlenwasserstoffgruppe; $-OR^{17}$;

wobei $R^{17}$ ausgewählt ist aus: Wasserstoff, unsubstituierte oder substituierte, lineare oder verzweigte, aliphatische oder aromatische Kohlenwasserstoffgruppe; wobei zwei oder mehr der $R^1$ bis $R^{17}$ kovalent miteinander verknüpft sein können;

wobei $M^1$ und $M^2$ ausgewählt ist aus: Alkalimetall, Erdalkalimetall, Ammonium, Phosphonium, und

wobei die Bedeutung für $M^1$ und $M^2$ unabhängig voneinander gewählt sein kann; wobei W für eine $P^{III}(G^2)(G^3)$-Gruppe steht:

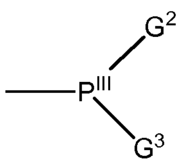

wobei $G^2$ und $G^3$ jeweils ausgewählt ist aus: Wasserstoff; lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; F, Cl, Br, I, oder $-OR^{18}$, $-C(O)R^{19}$, $-CO_2R^{20}$, $-CO_2M^1$, $-SR^{21}$, $-SOR^{22}$, $-SO_2R^{23}$, $-SO_3R^{24}$, $-SO_3M^2$, $-NR^{25}R^{26}$;

wobei $R^{18}$, $R^{19}$, $R^{20}$, $R^{21}$, $R^{22}$, $R^{23}$, $R^{24}$, $R^{25}$, $R^{26}$ ausgewählt sind aus: Wasserstoff, unsubstituierte oder substituierte, lineare oder verzweigte, aliphatische oder aromatische Kohlenwasserstoffgruppe; $-OR^{27}$;
wobei $R^{27}$ ausgewählt ist aus: Wasserstoff, unsubstituierte oder substituierte, lineare oder verzweigte, aliphatische oder aromatische Kohlenwasserstoffgruppe; F, Cl, Br, I;
wobei $M^1$ und $M^2$ ausgewählt ist aus: Alkalimetall, Erdalkalimetall, Ammonium, Phosphonium, und
wobei die Bedeutung für $M^1$ und $M^2$ unabhängig voneinander gewählt sein kann,
wobei die Bedeutung für $G^2$ und $G^3$ unabhängig voneinander gewählt sein kann, und $G^2$ und $G^3$ kovalent miteinander verknüpft sind,
wobei $G^5$ und $G^6$ ausgewählt ist aus: Wasserstoff; lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; F, Cl, Br, I, oder $-OR^{41}$, $-C(O)R^{42}$, $-CO_2R^{43}$, $-CO_2M^1$, $-SR^{44}$, $-SOR^{45}$, $-SO_2R^{46}$, $-SO_3R^{47}$, $-SO_3M^2$, $-NR^{48}R^{49}$,
wobei $R^{41}$, $R^{42}$, $R^{43}$, $R^{44}$, $R^{45}$, $R^{46}$, $R^{47}$, $R^{48}$, $R^{49}$ ausgewählt sind aus: Wasserstoff, unsubstituierte oder substituierte, lineare oder verzweigte, aliphatische oder aromatische Kohlenwasserstoffgruppe; $-OR^{50}$;
wobei $R^{50}$ ausgewählt ist aus: Wasserstoff, unsubstituierte oder substituierte, lineare oder verzweigte, aliphatische oder aromatische Kohlenwasserstoffgruppe;
wobei $M^1$ und $M^2$ ausgewählt ist aus: Alkalimetall, Erdalkalimetall, Ammonium, Phosphonium, und
wobei die Bedeutung für $M^1$ und $M^2$ unabhängig voneinander gewählt sein kann,
wobei die Bedeutung für $G^5$ und $G^6$ unabhängig voneinander gewählt sein kann, und $G^5$ und $G^6$ kovalent miteinander verknüpft sein können.
wobei $G^1$ das Strukturelement (V) umfasst:

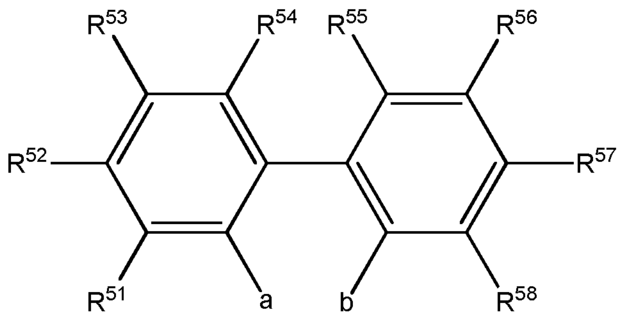

(V)

mit $R^{51}$, $R^{52}$, $R^{53}$, $R^{54}$, $R^{55}$, $R^{56}$, $R^{57}$, $R^{58}$ = Wasserstoff; lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; F, Cl, Br, oder I; oder $-OR^{59}$, $-COR^{60}$, $-CO_2R^{61}$,$-CO_2M^1$, $-SR^{62}$, $-SOR^{63}$, $-SO_2R^{64}$, $-SO_3R^{65}$, $-SO_3M^2$, $-NR^{66}R^{67}$, oder $N{=}CR^{68}R^{69}$; wobei die Bedeutung für jedes $R^{51}$ bis $R^{58}$ unabhängig voneinander gewählt sein kann und wobei zwei oder mehr der $R^{51}$ bis $R^{58}$ kovalent miteinander verknüpft sein können; wobei $R^{59}$, $R^{60}$, $R^{61}$, $R^{62}$, $R^{63}$, $R^{64}$, $R^{65}$, $R^{66}$, $R^{67}$ ausgewählt sind aus: Wasserstoff, unsubstituierte oder substituierte, lineare oder verzweigte, aliphatische oder aromatische Kohlenwasserstoffgruppe; $-OR^{68}$;
wobei $R^{68}$ ausgewählt ist aus: Wasserstoff, unsubstituierte oder substituierte, lineare oder verzweigte, aliphatische oder aromatische Kohlenwasserstoffgruppe;
wobei $M^1$ und $M^2$ ausgewählt ist aus: Alkalimetall, Erdalkalimetall, Ammonium, Phosphonium, und
wobei die Bedeutung für $M^1$ und $M^2$ unabhängig voneinander gewählt sein kann, und mit a und b als Anknüpfpunkte an $X^1$ und $X^2$.

In einer Ausführungsform ist $G^2$, $G^3$ = $-OR^{18}$.

In einer Ausführungsform ist $G^5$, $G^6$ = $-OR^{41}$.

In einer Ausführungsform weist die Verknüpfungen $G^2$-$G^3$ folgendes Strukturelement (VI) auf:

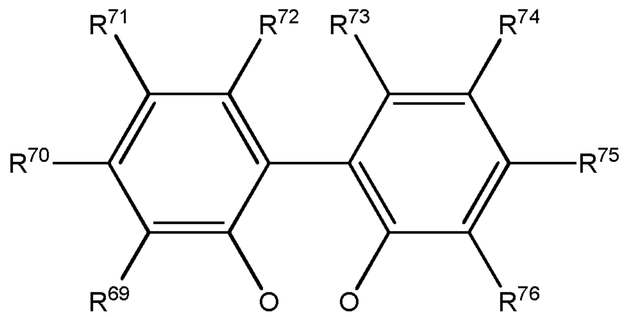

(VI)

mit $R^{69}$, $R^{70}$, $R^{71}$, $R^{72}$, $R^{73}$, $R^{74}$, $R^{75}$, $R^{76}$ = Wasserstoff; lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; F, Cl, Br, oder I; oder $-OR^{77}$, $-COR^{78}$, $-CO_2R^{79}$, $-CO_2M^1$, $-SR^{80}$, $-SOR^{81}$, $-SO_2R^{82}$, $-SO_3R^{83}$, $-SO_3M^2$, $-NR^{84}R^{85}$, oder $N=CR^{86}R^{87}$; wobei die Bedeutung für jedes $R^{69}$ bis $R^{76}$ unabhängig voneinander gewählt sein kann und wobei zwei oder mehr der $R^{69}$ bis $R^{76}$ kovalent miteinander verknüpft sein können;

wobei $R^{77}$, $R^{78}$, $R^{79}$, $R^{80}$, $R^{81}$, $R^{82}$, $R^{83}$, $R^{84}$, $R^{85}$ ausgewählt sind aus: Wasserstoff, unsubstituierte oder substituierte, lineare oder verzweigte, aliphatische oder aromatische Kohlenwasserstoffgruppe; $-OR^{86}$;

wobei $R^{86}$ ausgewählt ist aus: Wasserstoff, unsubstituierte oder substituierte, lineare oder verzweigte, aliphatische oder aromatische Kohlenwasserstoffgruppe;

wobei $M^1$ und $M^2$ ausgewählt ist aus: Alkalimetall, Erdalkalimetall, Ammonium, Phosphonium, und

wobei die Bedeutung für $M^1$ und $M^2$ unabhängig voneinander gewählt sein kann.

In einer Ausführungsform sind $G^5$ und $G^6$ kovalent miteinander verknüpft.

In einer Ausführungsform weist die Verknüpfungen $G^5$-$G^6$ folgendes Strukturelement (VII) auf:

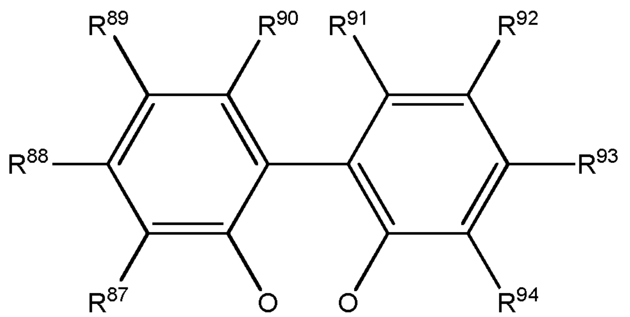

(VII)

mit $R^{87}$, $R^{88}$, $R^{89}$, $R^{90}$, $R^{91}$, $R^{92}$, $R^{93}$, $R^{94}$ = Wasserstoff; lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; F, Cl, Br, oder I; oder-$OR^{95}$, $-COR^{96}$, $-CO_2R^{97}$, $-CO_2M^1$, $-SR^{98}$, $-SOR^{99}$, $-SO_2R^{100}$, $-SO_3R^{101}$,$-SO_3M^2$, $-NR^{102}R^{103}$, oder $N=CR^{104}R^{105}$; wobei die Bedeutung für jedes $R^{31}$ bis $R^{38}$ unabhängig voneinander gewählt sein kann und wobei zwei oder mehr der $R^{86}$ bis $R^{93}$ kovalent miteinander verknüpft sein können;

wobei $R^{95}$, $R^{96}$, $R^{97}$, $R^{98}$, $R^{99}$, $R^{100}$, $R^{101}$, $R^{102}$, $R^{103}$ ausgewählt sind aus: Wasserstoff, unsubstituierte oder substituierte, lineare oder verzweigte, aliphatische oder aromatische Kohlenwasserstoffgruppe; $-OR^{104}$;

wobei $R^{104}$ ausgewählt ist aus: Wasserstoff, unsubstituierte oder substituierte, lineare oder verzweigte, aliphatische oder aromatische Kohlenwasserstoffgruppe; wobei $M^1$ und $M^2$ ausgewählt ist aus: Alkalimetall, Erdalkalimetall, Ammonium, Phosphonium, und

wobei die Bedeutung für $M^1$ und $M^2$ unabhängig voneinander gewählt sein kann.

In einer Ausführungsform entspricht die $P^{III}(G^2)(G^3)$-Gruppe von der Strukturformel der $P^{III}(G^5)(G^6)$-Gruppe.

Neben der Verbindung an sich wir auch Komplexverbindungen beansprucht, welche eine solche Verbindung umfassen.

In einer Ausführungsform der Erfindung umfasst die Komplexverbindung eine Verbindung wie sie zuvor beschrieben wurde und mindestens ein Metall-Zentralatom, wobei die Verbindung über mindestens ein $P^{III}$ an das Metall-Zentralatom koordiniert ist.

In einer Ausführungsform der Erfindung ist das Metall-Zentralatom ausgewählt aus den Gruppen 8 bis 10 des

Periodensystems der Elemente.

In einer bevorzugten Ausführungsform der Erfindung ist das Metall-Zentralatom Rhodium.

Neben der Komplexverbindung selbst wird auch eine Zusammensetzung beansprucht, welche eine solche Komplexverbindung umfasst.

In einer Ausführungsform enthält die Zusammensetzung eine Verbindung, wie sie zuvor beschrieben wurde, die nicht an ein Metall-Zentralatom koordiniert ist, und eine Komplexverbindung, wie sie zuvor beschrieben wurde.

Neben der Zusammensetzung wird auch die Verwendung eines solchen beansprucht.

In einer Ausführungsform wird die Zusammensetzung als katalytisch aktive Zusammensetzung in der Synthese organischer Verbindungen verwendet.

In einer Ausführungsform wird die Zusammensetzung als katalytisch aktive Zusammensetzung in einem Verfahren zur Hydroformylierung von olefinisch ungesättigten Kohlenwasserstoffgemischen verwendet.

Des Weiteren wird ein mehrphasiges Reaktionsgemisch beansprucht.

In einer Ausführungsform enthält das mehrphasige Reaktionsgemisch ein olefinisch ungesättigtes Kohlenwasserstoffgemisch, ein Gasgemisch enthaltend Kohlenmonoxid und Wasserstoff, Aldehyde, eine Zusammensetzung, wie sie zuvor beschrieben wurde, als katalytische aktive Zusammensetzung.

Des Weiteren wird auch ein Verfahren zur Hydroformylierung von olefinisch ungesättigten Kohlenwasserstoffgemischen zu Aldehyden beansprucht.
In einer Variante umfasst dieses Verfahren die folgenden Verfahrensschritte:

a) Bereitstellen eines Gemisches olefinisch ungesättigter Kohlenwasserstoffe;
b) Zugabe einer wie zuvor beschriebenen katalytisch aktiven Zusammensetzung;
c) Einleiten eines Gemisches, welches Kohlenmonoxid und Wasserstoff aufweist;
d) Erwärmen des Reaktionsgemisches auf eine Temperatur im Bereich von 80 bis 120 °C;
e) Aufbau eines Drucks im Bereich von 1,0 bis 6,4 MPa;
f) nach Abschluß der Reaktion Abtrennung des olefinisch ungesättigten Kohlenwasserstoffgemisches.

In einer Variante des Verfahrens umfasst dieses als zusätzlichen Verfahrenschritt:

g) Abtrennung und Rückführung von unumgesetzten, olefinisch ungesättigten Kohlenwasserstoffgemisch in den Verfahrensschritt a).

In einer Variante des Verfahrens umfasst dieses als zusätzlichen Verfahrenschritt:

h) Abtrennung und Rückführung der zur beschriebenen katalytisch aktiven Zusammensetzung in den Verfahrensschritt b).

In einer Variante des Verfahrens umfasst dieses als zusätzlichen Verfahrenschritt:

i) Abtrennung und Rückführung des unumgesetzten Gasgemischs, enthaltend Kohlenmonoxid und Wasserstoff in den Verfahrenschritt c).

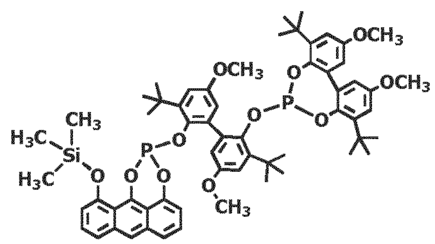

**6**

Ausführungsbeispiele von erfindungsgemäßen Tridentat-Verbindungen mit drei Phosphoratomen:

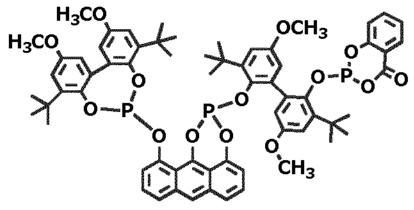

7

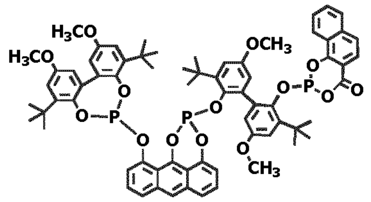

8

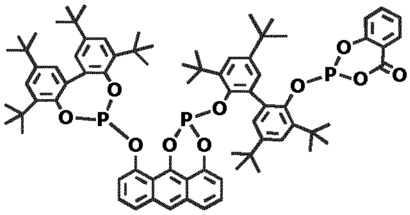

9

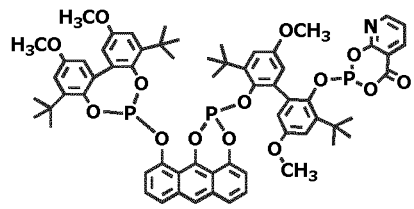

10

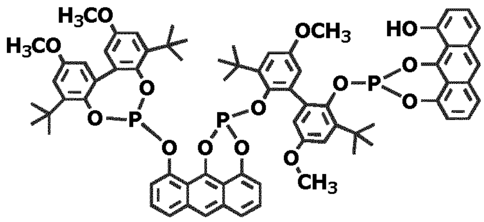

11

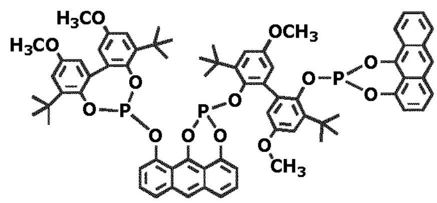

12

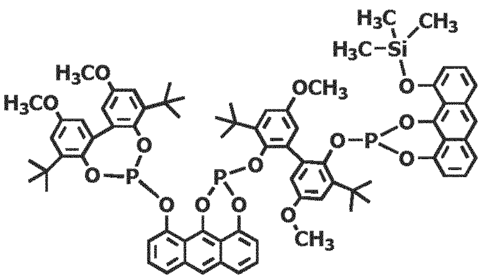

13

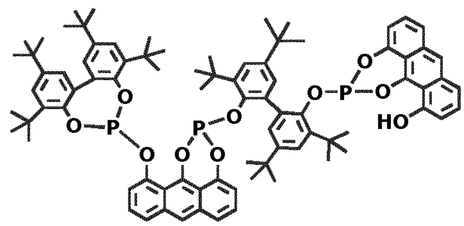

14

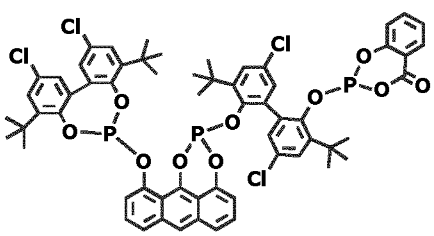

15

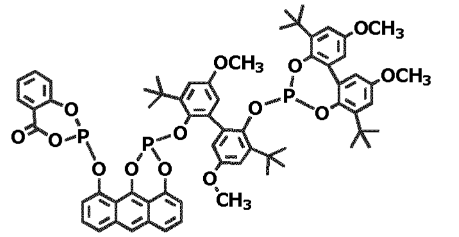

16

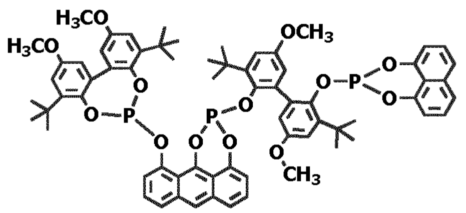

17

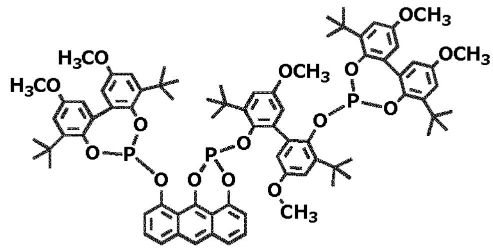

18

Synthesevorschriften ausgewählter Verbindungen

**Verbindung 6**

Eine Lösung aus **5** (0,994 g; 0,995 mmol) in THF (7 ml) wird mit Hexamethyldisilazan (0,802 g; 4,98 mmol), gelöst in THF (12 ml), versetzt. Die Reaktionslösung wird 10 h unter Rückfluss erhitzt und danach im Vakuum zur Trockne eingeengt. Der erhaltene Feststoff wird 2 h bei 50 °C/ 0,1 KPa getrocknet. Der Rückstand wird aus Hexan umkristallisiert. Ausbeute: 0,877 g (0,819 mmol; 82 %). $^1$H-NMR ($CD_2Cl_2$): δ 0,15-1,31 (45 H); 3,62-3,81 (12 H); 6,17-7,94 (m, 15 H) ppm.

**Verbindung 7**

Eine Lösung von **1** (0,966 g; 0,967 mmol) in Toluol (12 ml) wird bei Raumtemperatur unter Rühren mit Triethylamin (0,42 ml; 3,035 mmol) und anschließend bei 0°C mit einer Lösung von 2-Chloro-4H-benzo[*d*][1,3,2]dioxaphosphinin-4-on ( 0,196 g; 0,967 mmol) in Toluol (4 ml) versetzt. Man lässt auf Raumtemperatur erwärmen, rührt über Nacht und filtriert. Das Filtrat wird im Vakuum zur Trockne eingeengt, der Rückstand 3 h bei 40° C / 0,1 KPa getrocknet und dann säulenchromatografisch gereinigt (Laufmittel Hexan/ Dichlormethan, 1:10, $R_f$= 0,8). Ausbeute: 1,095 g (0,939 mmol, 97 %) Elementaranalyse (ber. für $C_{65}H_{67}O_{14}P_3$= 1165,15 g/mol): C 67,50 (67,01); H 5,80 (5,80); P 8,04 (7,97) %. $^1$H-NMR ($CD_2Cl_2$): 8 □ 1,08-1,65 (36 H); 3,68-3,94 (12 H); 6,10-8,10 (19 H) ppm. ESI-TOF HRMS (MeOH/ 0.1% HCOOH in $H_2O$ 90:10) m/e 1187.3633 (100%, M+Na)$^+$.

**Verbindung 8**

Eine Lösung von **1** (2,0 g; 2,002 mmol) in Toluol (20 ml) wird bei Raumtemperatur unter Rühren mit Triethylamin (0,88 ml; 6,314 mmol) und anschließend bei 0°C mit einer Lösung von 2-Chloro-4H-naphtho[1,2-*d*][1,3,2]dioxaphosphinin-4-one ( 0,656 mg; 2,602 mmol) in Toluol (7 ml) versetzt. Man lässt auf Raumtemperatur erwärmen, rührt über Nacht und filtriert. Das Filtrat wird im Vakuum zur Trockne eingeengt, der Rückstand 1 h bei 50° C / 0,1KPa getrocknet und dann säulenchromatografisch gereinigt (Laufmittel Hexan/ Dichlormethan, 1:10, $R_f$= 0,62). Ausbeute: 2,07 g (1,703 mmol; 85 %). Elementaranalyse (ber. für $C_{69}H_{69}O_{14}P_3$= 1215,21 g/mol): C 68,05 (68,20); H 5,85 (5,72); P 7,27 (7,65) %. $^1$H-NMR ($CD_2Cl_2$): δ 1,09-1,65 (36 H); 3,66-3,96 (12 H), 6,11-8,24 (21 H) ppm. CI-MS (Isobutan, pos.): m/e 1214 (1 %, M$^+$), 1044.

**Verbindung 9**

Eine Lösung von 2 (1,329 g; 1,204 mmol) in Toluol (15 ml) wird bei Raumtemperatur unter Rühren mit Triethylamin (0,53 ml; 3,781 mmol) und anschließend bei 0°C mit einer Lösung von 2-Chloro-4H-benzo[*d*][1,3,2]dioxaphosphinin-4-on ( 0,243 mg; 1,204 mmol) in Toluol (5 ml) versetzt. Man lässt auf Raumtemperatur erwärmen, rührt 48 h und filtriert. Das Filtrat wird im Vakuum zur Trockne eingeengt, der Rückstand 1 h bei 50° C / 0,1 KPa getrocknet und dann säulenchromatografisch gereinigt (Laufmittel Hexan/ Dichlormethan, 2:1, $R_f$= 0,22). Ausbeute: 1,14 g (0,898 mmol; 74 %). Elementaranalyse (ber. für $C_{77}H_{91}O_{10}P_3$= 1269,48 g/mol): C 73,07 (72,85); H 7,25 (7,23); P 7,37 (7,32) %. $^{31}$P-NMR ($CD_2Cl_2$): δ 102,5-103,7 (1 P); 118,5-119,8 (1 P); 135,6-136,3 (1 P) ppm. EI-MS: m/e 1268 (38 %, M$^+$-H), 1085 (43 %).

**Verbindung 10**

a) Chlorophosphit aus 2-Hydroxynicotinsäure, 2-Chloro-4H-[1,3,2]dioxaphosphinino[4,5-b]pyridin-4-on

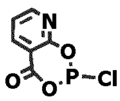

Eine Lösung von 2-Hydroxynicotionsäure (0,5 g; 3,594 mmol) und Triethylamin (1,5 ml; 10,783 mmol) in THF (20 ml) wird unter Rühren mit $PCl3$ (0,494 g; 3.594 mmol), gelöst in THF (8 ml) bei -20 °C, versetzt. Nach Rühren über Nacht bei Raumtemperatur und 2 h bei 70 °C wird die Reaktionslösung filtriert und der Feststoff mit THF (5 ml) gewaschen. Das Filtrat wird im Vakuum zur Trockne eingeengt und der gelbe Rückstand 1 h bei 50 °C / 0,1 KPa getrocknet. Ausbeute: 0,519 g (2.550 mmol; 71%). Der Feststoff besitzt laut NMR-Spketroskopie eine Reinheit von 95 mol-% und wurde ohne weitere Reinigung im nächsten Syntheseschritt eingesetzt.
$^1$H-NMR ($CD_2Cl_2$): δ□ 7,37 (dd, 1H); 8,40 (dd, 1H); 8,53 (dd, 1H) ppm.

b) Umsetzung zu **Verbindung 10**

Eine Lösung von **1** (1,859 g; 1,861 mmol) in Toluol (22 ml) wird bei Raumtemperatur unter Rühren mit Triethylamin (0,82 ml; 5,869 mmol) und anschließend bei 0°C mit einer Lösung von 2-Chloro-4H-[1,3,2]dioxaphosphinino[4,5-*b*]pyridin-4-on (0,4544 g; 2,233 mmol) in Toluol (14 ml) versetzt. Man läßt auf Raumtemperatur erwärmen, rührt über Nacht, filtriert und wäscht den Filterkuchen mit THF (2x 4ml). Die vereinigten Filtrate werden im Vakuum zur Trockne eingeengt und 3 h bei 50° C / 1 mbar getrocknet. Der Rückstand wird über Nacht mit 50 ml Hexan verrührt. Man filtriert, destilliert das Lösungsmittel im Vakuum ab und trocknet die erhaltene Festsubstanz 5 h bei 70 °C/ 0,1 KPa. Ausbeute: 2,00 g (1,715 mmol, 92 %). Elementaranalyse (ber. für $C_{64}H_{66}O_{14}NP_3$= 1166,14 g/mol): C 64,48 (65,92); H 5,70 (5,70); P 7,98 (7,97); N 1,36 (1,20) $^1$H-NMR ($CD_2Cl_2$): δ 0,77-1,62 (36 H); 3,56-3,74 (12 H); 5,89-8,44 (18 H) ppm. EI-MS: m/e 1165 (13 %, $M^+$).

**Verbindung 11**

Eine Lösung von **22** (2,135 g; 1,941 mmol) in Toluol (18 ml) wird bei Raumtemperatur unter Rühren mit Triethylamin (1,08 ml; 7,765 mmol) und anschließend bei 0°C mit festem 1,8,9-Anthracentriol (0,439 g; 1,941 mmol) versetzt. Man lässt auf Raumtemperatur erwärmen, rührt über Nacht, filtriert, entfernt das Lösungsmittel im Vakuum und trocknet 5 h bei 50 °C / 0,1 KPa. Ausbeute: 2,35 g (1,875 mmol, 96 %). Elementaranalyse (ber. für $C_{72}H_{71}O_{14}P_3$= 1253,26 g/mol): C 69,18 (69,00); H 5,86 (5,71); P 7,34 (7,42) %. □$^1$H-NMR ($CD_2Cl_2$): δ 0,77-1,46 (36 H); 3,41-3,71 (12 H); 5,78-8,42 (22 H); 12,06+12,76 (1 H) ppm. EI-MS: m/e 1253 (2%, $M^+$), 999 (100%).

**Verbindung 12**

Eine Lösung von **22** (1,082 g; 0,983 mmol) in Toluol (10 ml) wird bei Raumtemperatur unter Rühren mit Triethylamin (0,55 ml; 3,934 mmol) und anschließend bei 0°C mit festem Anthracendiol-1,9 (0,207 g; 0,983 mmol) versetzt. Man lässt auf Raumtemperatur erwärmen, rührt über Nacht und anschließend 2 h bei 70 °C, filtriert, entfernt das Lösungsmittel im Vakuum und trocknet 4 h bei 60 °C / 0,1 KPa. Der Rückstand wird säulenchromatografisch gereingt (Dichlormethan/Hexan=1:1, Rf = 0,27). Ausbeute: 0.931 g (0.752 mmol, 76 %). Elementaranalyse (ber. für $C_{72}H_{71}O_{13}P_3$= 1237,26 g/mol): C 69,77 (69,90); H 5,93 (5,78); P 7,52 (7,51) %. □$^1$H-NMR ($CD_2Cl_2$): δ 0,90-1,65 □(36 H); 3,61-3,91 (12 H); 6,05-8,28 (23 H) ppm. EI-MS: m/e 1238 (11 %, $M^+$), 982 (43 %), 579 (100 %).

**Verbindung 13**

Eine Lösung aus **11** (0,674 g; 0,537 mmol) in THF (4 ml) wird tropfenweise mit einer Lösung aus Hexamethyldisilazan (0,433 g; 2,689 mmol) in THF (8 ml) versetzt, 14 h unter Rückfluss gekocht und dann zur Trockene eingeengt. Der Rückstand wird mittels Säulenchromatographie gereinigt (Eluent Hexan/Dichlormethan, 1:2, Rf = 0.47). Ausbeute: 0,482 g (0,364 mmol; 68 %). Elementaranalyse (ber. für $C_{75}H_{79}O_{14}P_3Si$= 1325.44 g/mol): C 67,59 (67.96); H 6,09 (6.01); P 6,89 (7,01); Si 2,15 (2,12) %. $^1$H-NMR ($CD_2Cl_2$): δ 0,00-1,53 (45 H); 3,20-3,75 (12 H); 5,88-7,91 (22 H) ppm. Laut NMR-Spektroskopie liegen zwei diastereomere Produkte vor. ESI/TOF-HRMS: m/e 1325,45076 $(M+H)^+$.

**Verbindung 14**

Eine Lösung aus **23** (0,47 g; 0,390 mmol) und Triethylamin (0,158g; 1,561 mmol) in Toluol (5 ml) wird bei 0 °C mit festem 1,8,9-Anthracentriol (0,088 g; 0,390 mmol) versetzt. Nach Rühren über Nacht bei Raumtemperatur und 2 h bei 70 °C wird die Reaktionslösung filtriert und das Filtrat im Vakuum zur Trockne eingeengt. Der Rückstand wird mittels

Säulenchromatographie gereinigt (Eluent Hexan/Dichlormethan, 2:1, Rf = 0.4) Ausbeute: 0,270 g (0,199 mmol; 51 %). Elementaranalyse (ber. Für $C_{84}H_{95}O_{10}P_3$= 1357.58 g/mol): C 74,30 (74,32); H 6,89 (7,05); P 6,80 (6,85) %. $^{31}$P-NMR ($CD_2Cl_2$): δ 103,2 (s, br); 104,2 (s, br); 104,4 (d, $J_{PP}$= 10Hz); 104,7 (s, br); 105,3 (s); 106,4 (d, $J_{PP}$= 10Hz); 135,6 (s, br); 136,0 (s, br); 136,3 (s, br) ppm. Laut NMR-Spektroskopie liegen drei diastereomere Produkte vor. ESI/TOF-HRMS: m/e 1357.62109 $(M+H)^+$.

**Verbindung 15**

Eine Lösung aus **3** (1,479 g; 1,455 mmol) und Triethylamin (0,462 g; 4,568 mmol) in Toluol (20 ml) wird unter Rühren mit einer Lösung aus 2-Chlor-4H-benzo[*d*][1,3,2]dioxaphosphinin-4-on (0,338 g; 1,673 mmol) in Toluol (10 ml) bei 0 °C versetzt. Nach Rühren über Nacht bei Raumtemperatur wird die Reaktionslösung filtriert und das Filtrat im Vakuum zur Trockne eingeengt. Der erhaltene Feststoff wird 2 h bei 50 °C/ 0,1 KPa getrocknet und zur Reinigung aus Acetonitril umkristallisiert. Ausbeute: 1,133 g (0,958 mmol; 66 %). Elementaranalyse (ber. für $C_{61}H_{55}O_{10}P_3Cl_4$= 1182.82 g/mol): C 61,49 (61,94); H 4,71 (4,69); P 7,85 (7,86) %. $^1$H-NMR ($CD_2Cl_2$): δ 0,82-1,46 (36 H); 5,98-7,94 (19 Harom); Laut NMR-Spektroskopie liegen sechs diastereomere Produkte vor. EI-MS: m/e 1182 (10 %, $M^+$).

**Verbindung 16**

Eine Lösung aus **5** (0,999 g; 1 mmol) in Toluol (12 ml) wird bei Raumtemperatur unter Rühren mit Triethylamin (0,53 ml; 3,781 mmol) und anschließend bei 0 °C mit einer Lösung aus 2-Chlor-4H-benzo[d][1,3,2]dioxaphosphinin-4-on (0,203 g; 1 mmol) in Toluol (4 ml) versetzt. Man läßt auf Raumtemperatur erwärmen, rührt über Nacht und filtriert. Das Filtrat wird im Vakuum zur Trockne eingeengt, der Rückstand 3 h bei 40 °C / 0,1 KPa getrocknet und dann säulenchromatographisch gereinigt (Laufmittel Hexan / Dichlormethan, 1:10, $R_f$ = 0,8). Ausbeute: 1,107 g (0,950 mmol, 95 %) Elementaranalyse (ber. für $C_{65}H_{67}O_{14}P_3$= 1165,15 g/mol): C 67,35 (67,01); H 5,80 (5,80); P 8,01 (7,97) %. ESI-TOF HRMS (MeOH/ 0.1% HCOOH in $H_2O$ 90:10) m/e 1187.3633 (100%, $M+Na)^+$.

**Verbindung 17**

Zu einer Lösung aus **1** (1,487 g, 1,489 mmol) und Triethylamin (0,472 g; 4,673 mmol) in Toluol (17 ml) wird bei 0 °C eine Lösung aus 2-Chlornaphtho[1,8-*de*][1,3,2]dioxaphosphinin (0,333 g; 1,489 mmol) in Toluol (10 ml) gegeben. Nach Rühren über Nacht bei Raumtemperatur wird die Reaktionslösung filtriert und das Filtrat im Vakuum zur Trockne eingeengt. Der erhaltene Feststoff wird 2 h bei 50 °C/ 0,1 KPa getrocknet und aus Acetonitril (20 ml) umkristallisiert. Ausbeute: 1,087 g (0,915 mmol; 61 %). Elementaranalyse (ber. für $C_{68}H_{69}O_{13}P_3$= 1187,20 g/mol): C 68,67 (68,80); H 5,90 (5,86); P 7,83 (7,83) %. $^1$H-NMR ($CD_2Cl_2$): δ 1,00-1,63 (36 H); 3,67-3,89 (12 H); 6,02-8,02 (21H) ppm. EI-MS: m/e 1187 (20 %, $M^+$).

**Verbindung 18**

Eine Lösung von **1** (1,289g; 1,289 mmol) in THF (12 ml) wird bei -20 °C mit einer äquimolaren Menge n-BuLi in Hexan (5 ml) versetzt. Man läßt auf Raumtemperatur erwärmen, rührt über Nacht und gibt die so erhaltene Mischung bei 0 °C zu einer Lösung von 4,8-Di-*tert*-butyl-6-chloro-2,10-dimethoxydibenzo[*d*,*f*][1,3,2]dioxa-phosphepin (0,545 g; 1,289 mmol) in THF (9 ml). Die Mischung wird 16 h bei Raumtemperatur gerührt und im Vakuum zur Trockne eingeengt. Der Rückstand wird mit Toluol (12 ml) verrührt, man filtriert, engt das Filtrat im Vakuum ein und trocknet 3 h bei 50 °/ 0,1 KPa. $^{31}$P-NMR ($CD_2Cl_2$): δ 102,8; 104,4; 106,6; 109,6; 132,8; 134,4; 134,9; 136,9; 143,4 ppm.

NMR-spektroskopische Untersuchung zur Stabilität

Ligand **17** und der bidentate Vergleichsligand Biphephos wurden jeweils in unbehandeltem Toluol-Ds gelöst, in ein NMR-Röhrchen überführt und verschlossen. Über einen Zeitraum von 32 Tagen wurde der Gehalt der Liganden NMR-spektroskopisch verfolgt.

Die Ergebnisse sind in Figur 1 dargestellt. Der Ligand **17** weist eine wesentlich höhere Stabilität als der Vergleichsligand Biphephos auf, wie in Figur 1 deutlich zu erkennen ist. So ist der Vergleichsligand Biphephos nach dem 32. Tag NMR-technisch nicht mehr nachweisbar, während der Ligand **17** mit einer Konzentration von 60 % gegenüber dem Anfangswert gemessen wird.

Aus dieser Stabilitätsuntersuchung für die jeweils freien Liganden **17** und Biphphos ist die Stabilität für eine entsprechende katalytisch aktive Zusammensetzung, wie z.B. aus den daraus gebildeten Rhodium-Komplexderivaten, direkt ableitbar. Das bedeutet für einen mit dieser katalytisch aktiven Zusammensetzungen betriebenen Hydroformylierungsprozess, dass die Standzeit einer katalytisch aktiven Zusammensetzung auf Basis des Liganden **17** deutlich verlängert, somit ökonomisch optimiert wird. Dies geschieht ohne das eine weitere stabilisierende Komponente, wie z.B. die Zugabe von sterisch anspruchsvollen Aminderivaten - offenbart in EP 2280920 -, notwendig wird. Die nachfolgenden Katalyseversuche mit unterschiedlichen Olefinen bzw. unterschiedlichen olefinhaltigen Kohlenwasserstoffströmen demonstrieren diese technische Lehre im Detail.

Belegstruktur des tridentaten Charakters

Von Ligand 17 konnte ein Rhodiumkomplex dargestellt und in X-Ray-geeignter Qualität isoliert werden. Die aus der X-Ray-Aufnahme abgeleitete Struktur ist im Folgenden dargestellt.

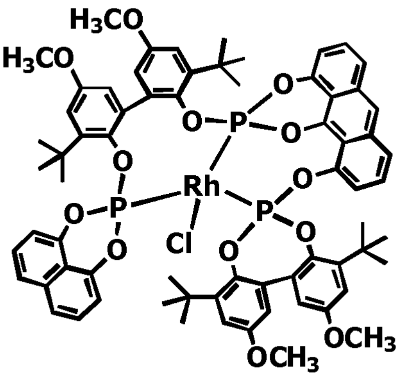

Die erhaltenen Daten belegen die 3-fach-Koordination des Rhodiums an $P^{III}$. Damit befindet sich in Lösung eine potentiell höhere $P^{III}$-Konzentration am Übergangsmetall mit der Folge, dass:

- Rhodium besser in Lösung, somit in Form der katalytisch aktiven Zusammensetzung, gehalten und
- die literaturbekannte Cluster-Bildung von Rhodium unterdrückt wird.

Ligandendissoziation und Cluster-Bildung sind weniger bevorzugt als bei Bidentat-Systemen, somit wird eine längere Standzeit der katalytisch aktiven Zusammensetzung ermöglicht.

Belegstruktur des tridentaten Charakters in Form einer Zweikernstruktur

Von Ligand 17 konnte ein Rhodiumkomplex dargestellt und in X-Ray-geeignter Qualität isoliert werden. Die aus der X-Ray-Aufnahme abgeleitete Struktur ist im Folgenden dargestellt:

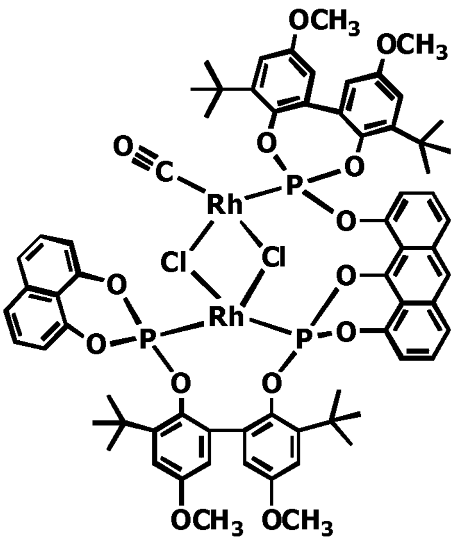

Die erhaltenen Daten belegen zu dem die Struktur eines zweikernigen Rhodiumkomplexes in der katalytisch aktiven Zusammensetzung. Die Stabilisierung eines zweiten Rhodiumatoms pro Komplex in der katalytisch aktiven

Zusammensetzung ist somit bewiesen und beugt auf diese Weise zusätzlich einer Cluster-Bildung, d.h. einem Verlust von Rhodium, vor.

Von den eingangs gestellten Anforderungen an neue Liganden werden die Punkte:

- verbesserte Beständigkeit gegenüber inherenten Katalysatorgiften sowie
- Unterdrückung der Rhodium-Cluster-Bildung durch Mehrfachkoordination mittels tridentater Liganden und Ausbildung zweikerniger Komplexe durch die Bereitstellung der erfindungsgemäßen Verbindungen und deren Verwendung als Liganden erfüllt.

Die Fähigkeit der erfindungsgemäßen Verbindungen in der Verwendung als Liganden in einer katalytisch aktiven Zusammensetzung isomerisierend zu hydroformylieren wird in den nachfolgenden Katalyseversuchen an Olefinen wie auch olefinhaltigen Gemischen offenbart.

<u>Arbeitsvorschrift für die Katalyseversuche</u>

Die Hydroformylierung wurde in einem mit Druckkonstanthaltung, Gasflussmessung, Begasungsrührer und Druckpipette ausgestatteten 200 ml-Autoklaven durchgeführt. Zur Minimierung eines Einflusses von Feuchtigkeit und Sauerstoff wurden sowohl die Lösungsmittel (Tol = Toluol, PC = Propylencarbonat, THF = Tetrahydrofuran), als auch die Substrate getrocknet. Für die Versuche wurden im Autoklaven unter Argonatmosphäre Lösungen des Rhodiums in Form von [(acac)Rh(COD)] (acac = Acetylacetonat-Anion; COD = 1,5-Cyclooctadien) als Katalysatorvorstufe in Toluol eingefüllt. Anschließend wurde die entsprechende Menge der in Toluol gelösten Phosphitverbindung, in der Regel 2 bis 5 Ligandäquivalente pro Rhodium, zugemischt. Die jeweils eingebrachte Masse an Toluol wurde bestimmt. Einwaagen der Olefine: 1-Octen (10,62 g; 94,64 mmol), n-Octene (10,70 g; 95,35 mmol), 2-Penten (2,81 g; 40,0 mmol, gekennzeichnet in untenstehender Tabelle mit ‚(P)', bzw. 9,75 g; 139,00 mmol. 1-Buten-, 2-Buten- und Isobuten-Zugaben erfolgen in analoger Weise Der Autoklav wurde bei einem Gesamtgasdruck (Synthesegas: $H_2$ (99,999%) : $CO_2$ (99,997%) = 1:1) von a) 4,2 MPa für einen Enddruck von 5,0 MPa; b) 1,2 MPa für den Enddruck von 2,0 MPa und c) 0,7 MPa für einen Enddruck von 1,0 MPa unter Rühren (1500 U/min) auf die jeweils angegebenen Temperaturen aufgeheizt. Nach Erreichen der Reaktionstemperatur wurde der Synthesegasdruck auf a) 4,85 MPa für einen Enddruck von 5,0 MPa, b) 1,95 MPa für einen Enddruck von 2,0 MPa und c) 0,95 MPa für einen Enddruck von 1,0 MPa erhöht das jeweils in der Tabelle angegebene Olefin oder olefinhaltige Gemisch mit einem in der Druckpipette eingestellten Überdruck von ca. 0,3 MPa zugepresst. Die Reaktion wurde bei konstantem Druck von jeweils 5,0, 2,0 bzw. 1,0 MPa über 4h geführt. Der Autoklav wurde nach Ablauf der Reaktionszeit auf Zimmertemperatur abgekühlt, unter Rühren entspannt und mit Argon gespült. Jeweils 1 ml der Reaktionsmischungen wurden unmittelbar nach Abschalten des Rührers entnommen, mit 5 ml Pentan verdünnt und gaschromatographisch analysiert: HP 5890 Series II plus, PONA, 50 m x 0,2 mm x 0,5 μm, Die quantitative Bestimmung von Restolefin und Aldehyd erfolgte gegen das Lösungsmittel Toluol als internen Standard.

Katalyseversuche mit Verbindungen **6** bis **17.**

Ausb. = Ausbeute bezogen auf das eingesetzte Olefin oder olefinhaltige Gemisch;

$$\text{Sel. (\%)} = \text{n-Selektivität (\%)}$$

<u>1-Octen</u>

| Ligand | P (MPa) | T(°C) | t (h) | [Rh] (ppm) | L/Rh | Solvens | Ausb. (%) | Sel. (%) |
|---|---|---|---|---|---|---|---|---|
| **7** | 5,0 | 100 | 4 | 40 | 1 | Tol | 85 | 95,8 |
| **7** | 5,0 | 100 | 4 | 40 | 2 | Tol | 86 | 95,5 |
| **9** | 5,0 | 100 | 4 | 40 | 2 | Tol | 86 | 94,9 |
| **9** | 5,0 | 100 | 4 | 40 | 2 | PC | 84 | 95,0 |
| **10** | 5,0 | 100 | 4 | 40 | 2 | Tol | 87 | 95,6 |
| **15** | 5,0 | 100 | 4 | 40 | 2 | Tol | 86 | 96,1 |

(fortgesetzt)

| Ligand | P (MPa) | T(°C) | t (h) | [Rh] (ppm) | L/Rh | Solvens | Ausb. (%) | Sel. (%) |
|---|---|---|---|---|---|---|---|---|
| **11** | 5,0 | 100 | 4 | 40 | 2 | Tol | 90 | 97,2 |
| **16** | 5,0 | 100 | 4 | 40 | 2 | Tol | 90 | 91,0 |
| **17** | 5,0 | 100 | 4 | 40 | 4 | Tol | 91 | 89,5 |

Alle verwendeten Liganden sina riena und weisen in der Umsetzung gute is hervorragende Ausbeuten sowie jeweils hervorragende n-Selektivitäten auf. Die jeweiligen katalytisch aktiven Zusammensetzungen benötigen für diese Leistungen nur geringe Ligandenüberschüsse, wie das **L/Rh**-Verhältnis in der Tabelle zeigt.

n-Octene (Octenisomerengemisch aus 1-Octen: 3,3 %; *cis+trans*-2-Octen: 48,5 %; *cis+trans*-3-Octen: 29,2%; *cis+trans*-Octen-4: 16,4 %; gerüstisomere Octene: 2,6 %)

| Ligand | P (MPa) | T(°C) | t(h) | [Rh] (ppm) | L/Rh | Solvens | Ausb. (%) | Sel. (%) |
|---|---|---|---|---|---|---|---|---|
| **7** | 2,0 | 120 | 4 | 100 | 1 | Tol | 68 | 84,2 |
| **7** | 2,0 | 120 | 4 | 100 | 2 | Tol | 79 | 85,5 |
| **7** | 2,0 | 120 | 4 | 100 | 10 | Tol | 74 | 85,6 |
| **8** | 2,0 | 120 | 4 | 100 | 2 | PC | 85 | 87,1 |
| **17** | 2,0 | 120 | 4 | 100 | 2 | Tol | 76 | 84,4 |

Alle verwendeten Liganden sind tridentat und weisen in der Umsetzung gute bis hervorragende Ausbeuten sowie jeweils hervorragende n-Selektivitäten auf. Die jeweiligen katalytisch aktiven Zusammensetzungen benötigen für diese Leistungen nur geringe Ligandenüberschüsse, wie das L/Rh-Verhältnis in der Tabelle zeigt. Höhere Ligandenüberschüsse sind nicht notwendig, wie am Beispiel des Liganden 7 in der Tabelle deutlich wird.

2-Penten (15 ml, 2,41 M) (P) kennzeichnet geringeren 2-Penteneinsatz (s.o.)

| Ligand | P (MPa) | T (°C) | t(h) | [Rh] (ppm) | L/Rh | Solvens | Ausb. (%) | Sel. (%) |
|---|---|---|---|---|---|---|---|---|
| **7** | 2,0 | 120 | 4 | 100 | 1 | Tol | 93 (P) | 89,6 |
| **7** | 2,0 | 120 | 4 | 100 | 1 | PC | 87 (P) | 91,6 |
| **7** | 2,0 | 120 | 4 | 100 | 2 | Tol | 95 (P) | 90,2 |
| **7** | 2,0 | 120 | 4 | 100 | 2 | PC | 93 (P) | 92,4 |
| **7** | 2,0 | 120 | 4 | 100 | 2 | PC | 90 | 92,2 |
| **7** | 2,0 | 120 | 4 | 100 | 5 | Tol | 95 | 90,0 |
| **7** | 2,0 | 120 | 4 | 100 | 10 | Tol | 95 (P) | 90,4 |
| **7** | 2,0 | 120 | 4 | 100 | 2 | Tol | 94 | 89,7 |
| **7** | 2,0 | 120 | 4 | 120 | 1,7 | Tol | 96 | 89,9 |
| **7** | 2,0 | 120 | 4 | 100 | 2/ 2 TINUVIN® | Tol | 96 | 90,2 |
| **7** | 2,0 | 100 | 4 | 100 | 2 | PC | 89 | 91,7 |
| **7** | 2,0 | 100 | 4 | 100 | 2 | Tol | 91 | 90,5 |
| **7** | 2,0 | 120 | 4 | 100 | 2 | Tol | 94 | 89,7 |

(fortgesetzt)

| Ligand | P (MPa) | T (°C) | t(h) | [Rh] (ppm) | L/Rh | Solvens | Ausb. (%) | Sel. (%) |
|---|---|---|---|---|---|---|---|---|
| **8** | 2,0 | 120 | 4 | 100 | 2 | PC | 95 | 92,7 |
| **8** | 2,0 | 120 | 4 | 100 | 5 | PC | 92 | 92,6 |
| **8** | 2,0 | 120 | 4 | 100 | 10 | PC | 95 | 92,6 |
| **8** | 2,0 | 120 | 4 | 100 | 2 | Tol | 95 | 90,6 |
| **8** | 2,0 | 120 | 4 | 100 | 2 | THF | 94 | 91,0 |
| **8** | 1,0 | 120 | 4 | 100 | 2 | PC | 92 | 90,7 |
| **8** | 2,0 | 100 | 4 | 100 | 2 | PC | 93 | 92,0 |
| **8** | 2,0 | 110 | 4 | 100 | 2 | PC | 94 | 92,4 |
| **10** | 2,0 | 120 | 4 | 100 | 2 | PC | 92 | 92,6 |
| **10** | 2,0 | 120 | 4 | 100 | 2 | Tol | 95 | 90,2 |
| **10** | 2,0 | 120 | 4 | 100 | 5 | Tol | 95 | 90,2 |
| **10** | 2,0 | 100 | 4 | 100 | 2 | PC | 90 | 92,8 |
| **11** | 2,0 | 120 | 4 | 100 | 2 | Tol | 95 (P) | 93,8 |
| **11** | 2,0 | 120 | 4 | 100 | 2 | Tol | 96 | 93,9 |
| **11** | 2,0 | 120 | 4 | 100 | 2 | PC | 93 (P) | 94,3 |
| **11** | 2,0 | 120 | 4 | 100 | 1 | Tol | 91 (P) | 93,4 |
| **11** | 2,0 | 120 | 4 | 100 | 5 | Tol | 95 (P) | 94,4 |
| **11** | 1,0 | 120 | 4 | 100 | 2 | Tol | 96 | 93,6 |
| **11** | 1,0 | 110 | 4 | 100 | 2 | Tol | 91 | 94,1 |
| **11** | 2,0 | 120 | 4 | 100 | 2 | PC | 99 | 94,4 |
| **11** | 1,0 | 110 | 4 | 100 | 2 | PC | 94 | 95,1 |
| **11** | 1,0 | 100 | 4 | 100 | 2 | PC | 90 | 95,8 |
| **11** | 2,0 | 100 | 4 | 100 | 2 | PC | 87 | 93,9 |
| **12** | 2,0 | 120 | 4 | 100 | 2 | Tol | 97 | 90,4 |
| **12** | 2,0 | 120 | 4 | 100 | 2 | PC | 97 | 91,9 |
| **12** | 1,0 | 100 | 4 | 100 | 2 | PC | 87 | 94,2 |
| **13** | 2,0 | 120 | 4 | 100 | 2 | PC | 89 | 94,5 |
| **13** | 1,0 | 110 | 4 | 100 | 2 | PC | 84 | 95,6 |
| **13** | 1,0 | 110 | 4 | 100 | 2 | PC | 91 | 95,4 |
| **14** | 2,0 | 120 | 4 | 100 | 2 | Tol | 88 (P) | 89,7 |
| **15** | 2,0 | 120 | 4 | 100 | 2 | PC | 95 | 92,6 |
| **15** | 2,0 | 120 | 4 | 100 | 2 | Tol | 91 | 90,1 |
| **6** | 2,0 | 120 | 4 | 100 | 2 | Tol | 65 | 88,6 |
| **17** | 2,0 | 100 | 4 | 100 | 2 | Tol | 87 | 89,6 |
| **17** | 2,0 | 120 | 4 | 100 | 2 | Tol | 93 | 90,8 |
| **17** | 2,0 | 120 | 4 | 100 | 5 | Tol | 97 | 90,2 |
| **17** | 2,0 | 120 | 4 | 100 | 2 | PC | 97 | 91,8 |

Die ausführliche Reihe der Katalyseversuche mit 2-Penten weist 2 Besonderheiten gegenüber den anderen Versuchsreihen auf:

- mit Ligand **6** findet eine bidentate Verbindung Verwendung, wobei ein deutlicher Rückgang der Ausbeute im Vergleich zu den anderen verwendeten Liganden registriert wird;
- der tridentate Ligand **7** wird in einem Versuch zusammen mit einem sterisch anspruchsvollen Aminderivat - Markenbezeichnung TINUVIN® = Sebacinsäuredi-4(2,2,6,6-Tetramethylpiperidinyl)ester - zur Umsetzung gebracht, wobei keine verbesserten Ergebnisse hinsichtlich der Ausbeute wie auch der n-Selektivität erzielt werden.

C-4-Olefine

| Ligand | Substrat | P (MPa) | T (°C) | t (h) | [Rh] (ppm) | L/Rh | Solvens | Ausb. (%) | Sel. (%) |
|---|---|---|---|---|---|---|---|---|---|
| **17** | 2-Buten | 2,0 | 120 | 5 | 40 | 3.9 | Toluol | 93.8 | 90.2 |
| **17** | 1-Buten | 2,0 | 120 | 5 | 37 | 6.0 | Toluol | 82.2 | 87.8 |
| **17** | Isobuten | 2,0 | 100 | 5 | 38 | 6.0 | Toluol | 64.6 | 100 |

**Patentansprüche**

1. Verbindung,
welche das Strukturelement (IV) aufweist:

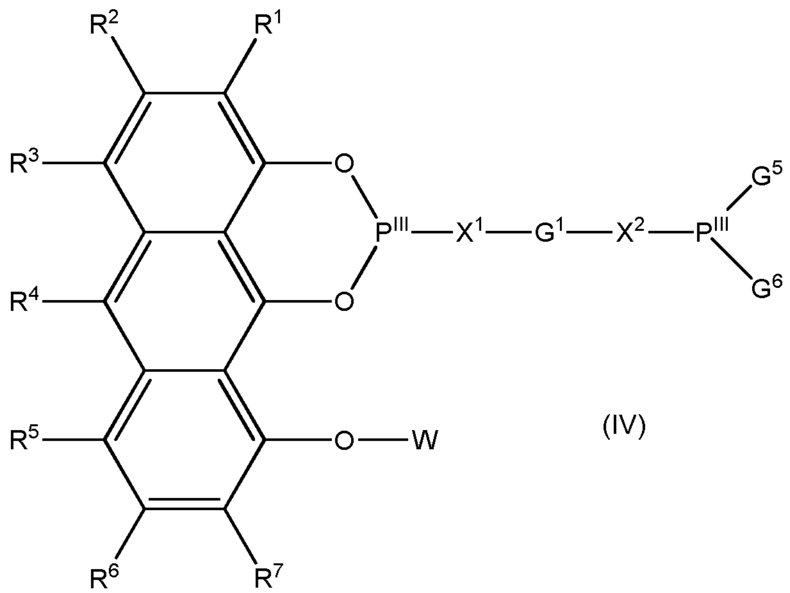

(IV)

mit $G^1$ = lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; wobei $X^1$, $X^2$ = O ist;
wobei $R^1$, $R^2$, $R^3$, $R^4$, $R^5$, $R^6$, $R^7$ ausgewählt sind aus: Wasserstoff, unsubstituierte oder substituierte, lineare oder verzweigte, aliphatische oder aromatische Kohlenwasserstoffgruppe; F, Cl, Br, I, $-OR^8$, $-C(O)R^9$, $-CO_2R^{10}$, $-CO_2M^1$, $-SR^{11}$,$-SOR^{12}$, $-SO_2R^{13}$, $-SO_3R^{14}$, $-SO_3M^2$, $-NR^{15}R^{16}$;
wobei $R^8$, $R^9$, $R^{10}$, $R^{11}$, $R^{12}$, $R^{13}$, $R^{14}$, $R^{15}$, $R^{16}$ ausgewählt sind aus: Wasserstoff, unsubstituierte oder substituierte, lineare oder verzweigte, aliphatische oder aromatische oder heteroaromatische oder kondensierte aromatische oder kondensierte aromatisch-heteroaromatische Kohlenwasserstoffgruppe; $-OR^{17}$;
wobei $R^{17}$ ausgewählt ist aus: Wasserstoff, unsubstituierte oder substituierte, lineare oder verzweigte, aliphatische oder aromatische Kohlenwasserstoffgruppe; wobei zwei oder mehr der $R^1$ bis $R^{17}$ kovalent miteinander verknüpft sein können;

wobei $M^1$ und $M^2$ ausgewählt ist aus: Alkalimetall, Erdalkalimetall, Ammonium, Phosphonium, und wobei die Bedeutung für $M^1$ und $M^2$ unabhängig voneinander gewählt sein kann; wobei W für eine $P^{III}(G^2)(G^3)$-Gruppe steht:

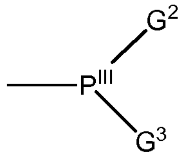

wobei $G^2$ und $G^3$ jeweils ausgewählt ist aus: Wasserstoff; lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; F, Cl, Br, I, oder $-OR^{18}$, $-C(O)R^{19}$, $-CO_2R^{20}$, $-CO_2M^1$, $-SR^{21}$, $-SOR^{22}$, $-SO_2R^{23}$, $-SO_3R^{24}$, $-SO_3M^2$, $-NR^{25}R^{26}$;
wobei $R^{18}$, $R^{19}$, $R^{20}$, $R^{21}$, $R^{22}$, $R^{23}$, $R^{24}$, $R^{25}$, $R^{26}$ ausgewählt sind aus: Wasserstoff, unsubstituierte oder substituierte, lineare oder verzweigte, aliphatische oder aromatische Kohlenwasserstoffgruppe; $-OR^{27}$;
wobei $R^{27}$ ausgewählt ist aus: Wasserstoff, unsubstituierte oder substituierte, lineare oder verzweigte, aliphatische oder aromatische Kohlenwasserstoffgruppe; F, Cl, Br, I; wobei $M^1$ und $M^2$ ausgewählt ist aus: Alkalimetall, Erdalkalimetall, Ammonium, Phosphonium, und
wobei die Bedeutung für $M^1$ und $M^2$ unabhängig voneinander gewählt sein kann, wobei die Bedeutung für $G^2$ und $G^3$ unabhängig voneinander gewählt sein kann, und $G^2$ und $G^3$ kovalent miteinander verknüpftsind,
wobei $G^5$ und $G^6$ ausgewählt ist aus: Wasserstoff; lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; F, Cl, Br, I, oder $-OR^{41}$, $-C(O)R^{42}$, $-CO_2R^{43}$, $-CO_2M^1$, $-SR^{44}$, $-SOR^{45}$, $-SO_2R^{46}$, $-SO_3R^{47}$, $-SO_3M^2$, $-NR^{48}R^{49}$,
wobei $R^{41}$, $R^{42}$, $R^{43}$, $R^{44}$, $R^{45}$, $R^{46}$, $R^{47}$, $R^{48}$, $R^{49}$ ausgewählt sind aus: Wasserstoff, unsubstituierte oder substituierte, lineare oder verzweigte, aliphatische oder aromatische Kohlenwasserstoffgruppe; $-OR^{50}$;
wobei $R^{50}$ ausgewählt ist aus: Wasserstoff, unsubstituierte oder substituierte, lineare oder verzweigte, aliphatische oder aromatische Kohlenwasserstoffgruppe;
wobei $M^1$ und $M^2$ ausgewählt ist aus: Alkalimetall, Erdalkalimetall, Ammonium, Phosphonium, und
wobei die Bedeutung für $M^1$ und $M^2$ unabhängig voneinander gewählt sein kann, wobei die Bedeutung für $G^5$ und $G^6$ unabhängig voneinander gewählt sein kann, und $G^5$ und $G^6$ kovalent miteinander verknüpft sein können.

wobei $G^1$ das Strukturelement (V) umfasst:

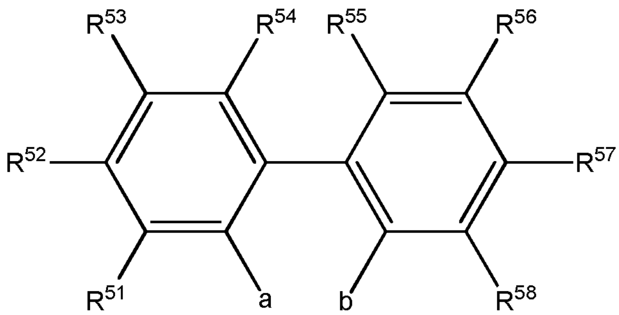

(V)

mit $R^{51}$, $R^{52}$, $R^{53}$, $R^{54}$, $R^{55}$, $R^{56}$, $R^{57}$, $R^{58}$ = Wasserstoff; lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; F, Cl, Br, oder I; oder $-OR^{59}$, $-COR^{60}$, $-CO_2R^{61}$, $-CO_2M^1$, $-SR^{62}$, $-SOR^{63}$, $-SO_2R^{64}$, $-SO_3R^{65}$, $-SO_3M^2$, $-NR^{66}R^{67}$, oder $N{=}CR^{68}R^{69}$; wobei die Bedeutung für jedes $R^{51}$ bis $R^{58}$ unabhängig voneinander gewählt sein kann und wobei zwei oder mehr der $R^{51}$ bis $R^{58}$ kovalent miteinander verknüpft sein können;
wobei $R^{59}$, $R^{60}$, $R^{61}$, $R^{62}$, $R^{63}$, $R^{64}$, $R^{65}$, $R^{66}$, $R^{67}$ ausgewählt sind aus: Wasserstoff, unsubstituierte oder substituierte, lineare oder verzweigte, aliphatische oder aromatische Kohlenwasserstoffgruppe; $-OR^{68}$;
wobei $R^{68}$ ausgewählt ist aus: Wasserstoff, unsubstituierte oder substituierte, lineare oder verzweigte, aliphatische oder aromatische Kohlenwasserstoffgruppe;

wobei $M^1$ und $M^2$ ausgewählt ist aus: Alkalimetall, Erdalkalimetall, Ammonium, Phosphonium, und wobei die Bedeutung für $M^1$ und $M^2$ unabhängig voneinander gewählt sein kann, und mit a und b als Anknüpfpunkte an $X^1$ und $X^2$.

**2.** Verbindung nach Anspruch 1, wobei $G^2$, $G^3$ = $-OR^{18}$ ist.

**3.** Verbindung nach einem der Ansprüche 1 oder 2, wobei $G^5$, $G^6$ = $-OR^{41}$ ist.

**4.** Verbindung nach einem der Ansprüche 1 bis 3, wobei die Verknüpfungen $G^2$-$G^3$ folgendes Strukturelement (VI) aufweist:

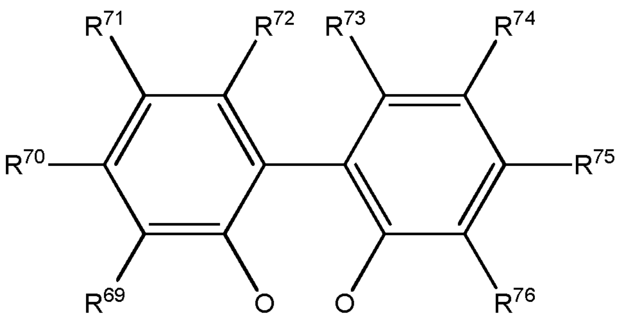

(VI)

mit $R^{69}$, $R^{70}$, $R^{71}$, $R^{72}$, $R^{73}$, $R^{74}$, $R^{75}$, $R^{76}$ = Wasserstoff; lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; F, Cl, Br, oder I; oder $-OR^{77}$, $-COR^{78}$, $-CO_2R^{79}$,$-CO_2M^1$, $-SR^{80}$, $-SOR^{81}$, $-SO_2R^{82}$, $-SO_3R^{83}$, $-SO_3M^2$, $-NR^{84}R^{85}$, oder $N=CR^{86}R^{87}$; wobei die Bedeutung für jedes $R^{69}$ bis $R^{76}$ unabhängig voneinander gewählt sein kann und wobei zwei oder mehr der $R^{69}$ bis $R^{76}$ kovalent miteinander verknüpft sein können; wobei $R^{77}$, $R^{78}$, $R^{79}$, $R^{80}$, $R^{81}$, $R^{82}$, $R^{83}$, $R^{84}$, $R^{85}$ ausgewählt sind aus: Wasserstoff, unsubstituierte oder substituierte, lineare oder verzweigte, aliphatische oder aromatische Kohlenwasserstoffgruppe; $-OR^{86}$;
wobei $R^{86}$ ausgewählt ist aus: Wasserstoff, unsubstituierte oder substituierte, lineare oder verzweigte, aliphatische oder aromatische Kohlenwasserstoffgruppe;
wobei $M^1$ und $M^2$ ausgewählt ist aus: Alkalimetall, Erdalkalimetall, Ammonium, Phosphonium, und wobei die Bedeutung für $M^1$ und $M^2$ unabhängig voneinander gewählt sein kann.

**5.** Verbindung nach einem der Ansprüche 1 bis 4, wobei $G^5$ und $G^6$ kovalent miteinander verknüpft sind.

**6.** Verbindung nach einem der Ansprüche 1 bis 5, wobei die Verknüpfungen $G^5$-$G^6$ folgendes Strukturelement (VII) aufweist:

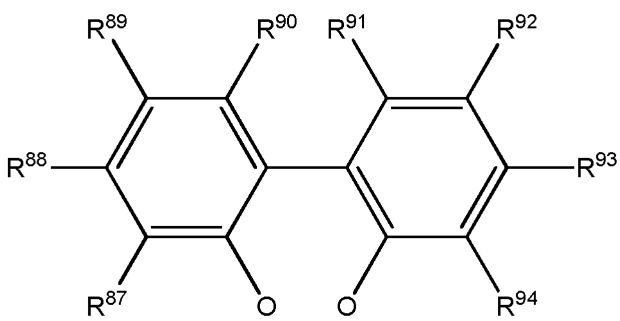

(VII)

mit $R^{87}$, $R^{88}$, $R^{89}$, $R^{90}$, $R^{91}$, $R^{92}$, $R^{93}$, $R^{94}$ = Wasserstoff; lineare oder verzweigte, aliphatische oder aromatische oder heteroaromatische oder kondensierte aromatische oder kondensierte aromatisch-heteroaromatische Kohlenwasserstoffgruppe mit beliebiger weiterer Substitution; F, Cl, Br, oder I; oder-$OR^{95}$, $-COR^{96}$, $-CO_2R^{97}$,$-CO_2M^1$, $-SR^{98}$, $-SOR^{99}$, $-SO_2R^{100}$, $-SO_3R^{101}$, $-SO_3M^2$, $-NR^{102}R^{103}$, oder $N=CR^{104}R^{105}$; wobei die

Bedeutung für jedes $R^{31}$ bis $R^{38}$ unabhängig voneinander gewählt sein kann und wobei zwei oder mehr der $R^{86}$ bis $R^{93}$ kovalent miteinander verknüpft sein können;
wobei $R^{95}$, $R^{96}$, $R^{97}$, $R^{98}$, $R^{99}$, $R^{100}$, $R^{101}$, $R^{102}$, $R^{103}$ ausgewählt sind aus: Wasserstoff, unsubstituierte oder substituierte, lineare oder verzweigte, aliphatische oder aromatische Kohlenwasserstoffgruppe; $-OR^{104}$;
wobei $R^{104}$ ausgewählt ist aus: Wasserstoff, unsubstituierte oder substituierte, lineare oder verzweigte, aliphatische oder aromatische Kohlenwasserstoffgruppe; wobei $M^1$ und $M^2$ ausgewählt ist aus: Alkalimetall, Erdalkalimetall, Ammonium, Phosphonium, und
wobei die Bedeutung für $M^1$ und $M^2$ unabhängig voneinander gewählt sein kann.

**7.** Verbindung nach einem der Ansprüche 1 bis 6,
wobei die $P^{III}(G^2)(G^3)$-Gruppe von der Strukturformel der $P^{III}(G^5)(G^6)$-Gruppe entspricht.

**8.** Komplexverbindung umfassend:

- eine Verbindung nach einem der Ansprüche 1 bis 7,
- mindestens ein Metall-Zentralatom,

wobei die Verbindung über mindestens ein $P^{III}$ an das Metall-Zentralatom koordiniert ist.

**9.** Komplexverbindung nach Anspruch 8,
wobei das Metall-Zentralatom ausgewählt ist aus den Gruppen 8 bis 10 des Periodensystems der Elemente.

**10.** Komplexverbindung nach Anspruch 9, wobei das Metall-Zentralatom Rhodium ist.

**11.** Zusammensetzung, enthaltend:

- eine Verbindung nach einem der Ansprüche 1 bis 7, die nicht an ein Metall-Zentralatom koordiniert ist, und
- eine Komplexverbindung nach einem der Ansprüche 8 bis 10.

**12.** Verwendung einer Zusammensetzung nach Anspruch 11 als katalytisch aktive Zusammensetzung in der Synthese organischer Verbindungen.

**13.** Verwendung einer Zusammensetzung nach Anspruch 11 als katalytisch aktive Zusammensetzung in einem Verfahren zur Hydroformylierung von olefinisch ungesättigten Kohlenwasserstoffgemischen.

**14.** Mehrphasiges Reaktionsgemisch, enthaltend:

- ein olefinisch ungesättigtes Kohlenwasserstoffgemisch,
- ein Gasgemisch enthaltend Kohlenmonoxid und Wasserstoff,
- Aldehyde,
- eine Zusammensetzung nach Anspruch 11 als katalytische aktive Zusammensetzung.

**15.** Verfahren zur Hydroformylierung von olefinisch ungesättigten Kohlenwasserstoffgemischen zu Aldehyden umfassend die Verfahrensschritte:

a) Bereitstellen eines Gemisches olefinisch ungesättigter Kohlenwasserstoffe;
b) Zugabe einer katalytisch aktiven Zusammensetzung gemäß Anspruch 11;
c) Einleiten eines Gemisches, welches Kohlenmonoxid und Wasserstoff aufweist;
d) Erwärmen des Reaktionsgemisches auf eine Temperatur im Bereich von 80 bis 120 °C;
e) Aufbau eines Drucks im Bereich von 1,0 bis 6,4 MPa;
f) nach Abschluß der Reaktion Abtrennung des olefinisch ungesättigten Kohlenwasserstoffgemisches.

**16.** Verfahren nach Anspruch 15,
umfassend einen weiteren Verfahrensschritt:

g) Abtrennung und Rückführung von unumgesetzten, olefinisch ungesättigten Kohlenwasserstoffgemisch in den Verfahrensschritt a).

**17.** Verfahren nach einem der Ansprüche 15 oder 16,

umfassend einen weiteren Verfahrensschritt:

h) Abtrennung und Rückführung der katalytisch aktiven Zusammensetzung gemäß Anspruch 14 in den Verfahrensschritt b).

**18.** Verfahren nach einem der Ansprüche 15 bis 17, umfassend einen weiteren Verfahrensschritt:

i) Abtrennung und Rückführung des unumgesetzten Gasgemischs, enthaltend Kohlenmonoxid und Wasserstoff in den Verfahrenschritt c).

### Claims

**1.** Compound, which comprises the structural element (IV):

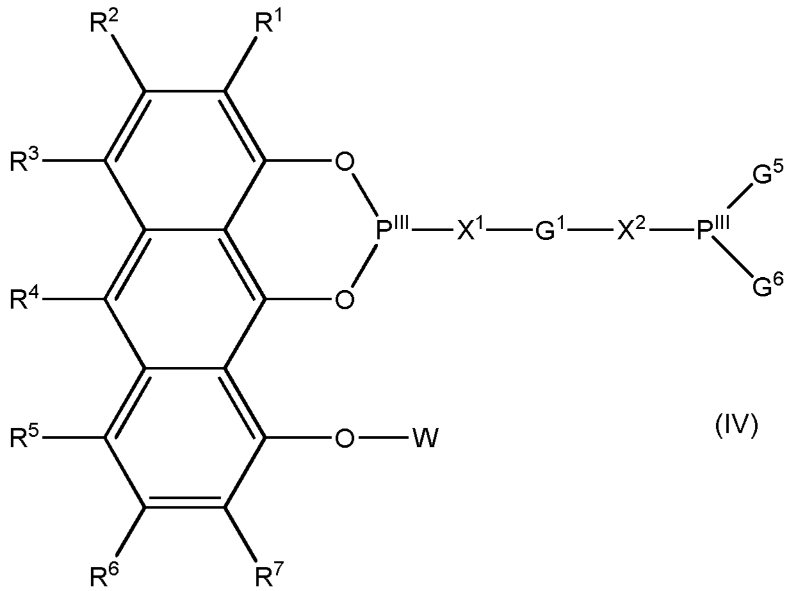

(IV)

with $G^1$ = a linear or branched, aliphatic or aromatic or heteroaromatic or fused aromatic or fused aromatic-heteroaromatic hydrocarbon group with any desired further substitution;
wherein $X^1$, $X^2$ = O
wherein $R^1$, $R^2$, $R^3$, $R^4$, $R^5$, $R^6$, $R^7$ are selected from: hydrogen, substituted or unsubstituted, linear or branched, aliphatic or aromatic hydrocarbon group; F, Cl, Br, I, $-OR^8$, $-C(O)R^9$, $-CO_2R^{10}$, $-CO_2M^1$, $-SR^{11}$, $-SOR^{12}$, $-SO_2R^{13}$, $-SO_3R^{14}$, $-SO_3M^2$, $-NR^{15}R^{16}$;
wherein $R^8$, $R^9$, $R^{10}$, $R^{11}$, $R^{12}$, $R^{13}$, $R^{14}$, $R^{15}$, $R^{16}$ are selected from: hydrogen, substituted or unsubstituted, linear or branched, aliphatic or aromatic or heteroaromatic or fused aromatic or fused aromatic-heteroaromatic hydrocarbon group; $-OR^{17}$;
wherein $R^{17}$ is selected from: hydrogen, unsubstituted or substituted, linear or branched, aliphatic or aromatic hydrocarbon group; wherein two or more of $R^1$ to $R^{17}$ may be linked to each other covalently;
wherein $M^1$ and $M^2$ are selected from: alkali metal, alkaline earth metal, ammonium, phosphonium, and wherein the meaning may have been chosen for $M^1$ and $M^2$ independently of each other;
wherein W represents a $P^{III}(G^2)$ $(G^3)$ group:

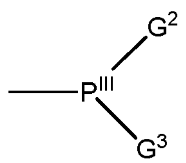

wherein $G^2$ and $G^3$ are each selected from: hydrogen; linear or branched, aliphatic or aromatic or heteroaromatic or fused aromatic or fused aromatic-heteroaromatic hydrocarbon group with any desired further substitution; F, Cl, Br, I, or $-OR^{18}$, $-C(O)R^{19}$, $-CO_2R^{20}$, $-CO_2M^1$, $-SR^{21}$,$-SOR^{22}$, $-SO_2R^{23}$, $-SO_3R^{24}$, $-SO_3M^2$, $-NR^{25}R^{26}$;
wherein $R^{18}$, $R^{19}$, $R^{20}$, $R^{21}$, $R^{22}$, $R^{23}$, $R^{24}$, $R^{25}$, $R^{26}$ are selected from: hydrogen, substituted or unsubstituted, linear or branched, aliphatic or aromatic hydrocarbon group; $-OR^{27}$; wherein $R^{27}$ is selected from: hydrogen, substituted or unsubstituted, linear or branched, aliphatic or aromatic hydrocarbon group; F, Cl, Br, I;
wherein $M^1$ and $M^2$ are selected from: alkali metal, alkaline earth metal, ammonium, phosphonium, and wherein the meaning may have been chosen for $M^1$ and $M^2$ independently of each other,
wherein the meaning may have been chosen for $G^2$ and $G^3$ independently of each other, and $G^2$ and $G^3$ are linked to each other covalently,
wherein $G^5$ and $G^6$ are selected from: hydrogen; linear or branched; aliphatic or aromatic or heteroaromatic or fused aromatic or fused aromatic-heteroaromatic hydrocarbon group with any desired further substitution; F, Cl, Br, I, or $-OR^{41}$, $-C(O)R^{42}$, $-CO_2R^{43}$, $-CO_2M^1$, $-SR^{44}$,$-SOR^{45}$, $-SO_2R^{46}$, $-SO_3R^{47}$, $-SO_3M^2$, $-NR^{48}R^{49}$,
wherein $R^{41}$, $R^{42}$, $R^{43}$, $R^{44}$, $R^{45}$, $R^{46}$, $R^{47}$, $R^{48}$, $R^{49}$ are selected from: hydrogen, substituted or unsubstituted, linear or branched, aliphatic or aromatic hydrocarbon group; $-OR^{50}$; wherein $R^{50}$ is selected from: hydrogen, substituted or unsubstituted, linear or branched, aliphatic or aromatic hydrocarbon group;
wherein $M^1$ and $M^2$ are selected from: alkali metal, alkaline earth metal, ammonium, phosphonium, and wherein the meaning may have been chosen for $M^1$ and $M^2$ independently of each other,
wherein the meaning may have been chosen for $G^5$ and $G^6$ independently of each other, and $G^5$ and $G^6$ may be linked to each other covalently,
wherein $G^1$ comprises the structural element (V):

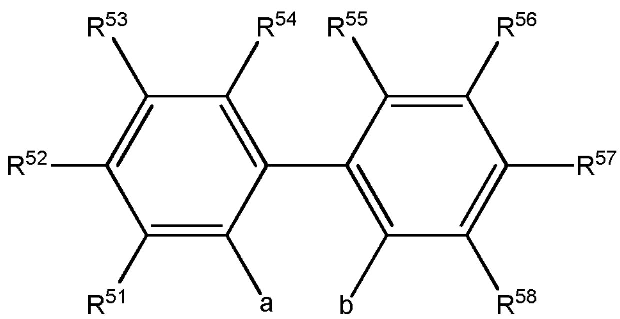

(V)

with $R^{51}$, $R^{52}$, $R^{53}$, $R^{54}$, $R^{55}$, $R^{56}$, $R^{57}$, $R^{58}$ = hydrogen; linear or branched, aliphatic or aromatic or heteroaromatic or fused aromatic or fused aromatic-heteroaromatic hydrocarbon group with any desired further substitution; F, Cl, Br, or I; or $-OR^{59}$, $-COR^{60}$, $-CO_2R^{61}$, $-CO_2M^1$, $-SR^{62}$,$-SOR^{63}$, $-SO_2R^{64}$, $-SO_3R^{65}$, $-SO_3M^2$, $-NR^{66}R^{67}$, or $N=CR^{68}R^{69}$; wherein the meaning may have been chosen independently for each $R^{51}$ to $R^{58}$ independently of each other and wherein two or more of $R^{51}$ to $R^{58}$ may be linked to each other covalently;
wherein $R^{59}$, $R^{60}$, $R^{61}$, $R^{62}$, $R^{63}$, $R^{64}$, $R^{65}$, $R^{66}$, $R^{67}$ are selected from: hydrogen, substituted or unsubstituted, linear or branched, aliphatic or aromatic hydrocarbon group; $-OR^{68}$; wherein $R^{68}$ is selected from: hydrogen, substituted or unsubstituted, linear or branched, aliphatic or aromatic hydrocarbon group;
wherein $M^1$ and $M^2$ are selected from: alkali metal, alkaline earth metal, ammonium, phosphonium, and wherein the meaning may have been chosen for $M^1$ and $M^2$ independently of each other,
and with a and b as attachment points to $X^1$ and $X^2$.

2. Compound according to Claim 1,
wherein $G^2$, $G^3$ is = $-OR^{18}$.

3. Compound according to either of Claims 1 and 2,
wherein $G^5$, $G^6$ is = $-OR^{41}$.

4. Compound according to any of Claims 1 to 3, wherein the links $G^2$-$G^3$ comprises the following structural element (VI):

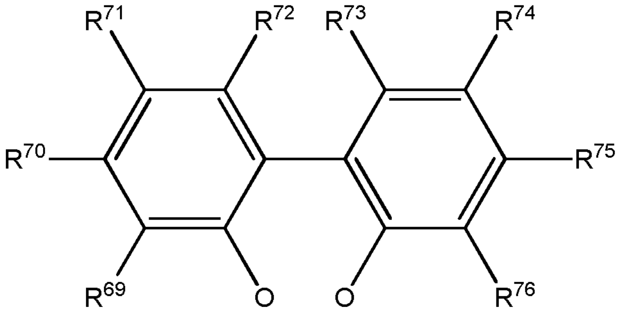

(VI)

with $R^{69}$, $R^{70}$, $R^{71}$, $R^{72}$, $R^{73}$, $R^{74}$, $R^{75}$, $R^{76}$ = hydrogen; linear or branched, aliphatic or aromatic or heteroaromatic or fused aromatic or fused aromatic-heteroaromatic hydrocarbon group with any desired further substitution; F, Cl, Br, or I; or $-OR^{77}$, $-COR^{78}$, $-CO_2R^{79}$, $-CO_2M^1$, $-SR^{80}$,$-SOR^{81}$, $-SO_2R^{82}$, $-SO_3R^{83}$, $-SO_3M^2$, $-NR^{84}R^{85}$, or $N=CR^{86}R^{87}$; wherein the meaning may have been chosen for each $R^{69}$ to $R^{76}$ independently of each other and wherein two or more of $R^{69}$ to $R^{76}$ may be linked to each other covalently;
wherein $R^{77}$, $R^{78}$, $R^{79}$, $R^{80}$, $R^{81}$, $R^{82}$, $R^{83}$, $R^{84}$, $R^{85}$ are selected from: hydrogen, substituted or unsubstituted, linear or branched, aliphatic or aromatic hydrocarbon group; $-OR^{86}$; wherein $R^{86}$ is selected from: hydrogen, substituted or unsubstituted, linear or branched, aliphatic or aromatic hydrocarbon group;
wherein $M^1$ and $M^2$ are selected from: alkali metal, alkaline earth metal, ammonium, phosphonium, and wherein the meaning may have been chosen for $M^1$ and $M^2$ independently of each other.

**5.** Compound according to any of Claims 1 to 4,
wherein $G^5$ and $G^6$ are linked to each other covalently.

**6.** Compound according to any of Claims 1 to 5, wherein the links $G^5$-$G^6$ comprises the following structural element (VII):

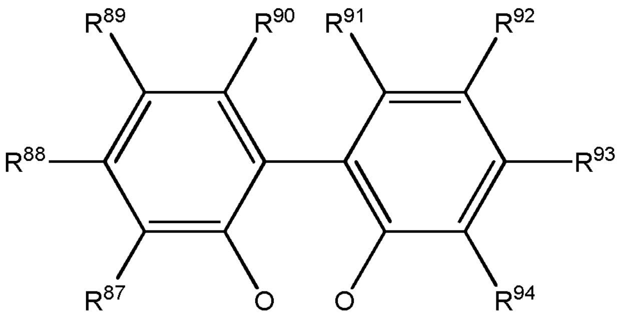

(VII)

with $R^{87}$, $R^{88}$, $R^{89}$, $R^{90}$, $R^{91}$, $R^{92}$, $R^{93}$, $R^{94}$ = hydrogen; linear or branched, aliphatic or aromatic or heteroaromatic or fused aromatic or fused aromatic-heteroaromatic hydrocarbon group with any desired further substitution; F, Cl, Br, or I; or $-OR^{95}$, $-COR^{96}$, $-CO_2R^{97}$, $-CO_2M^1$, $-SR^{98}$,$-SOR^{99}$, $-SO_2R^{100}$, $-SO_3R^{101}$, $-SO_3M^2$, $-NR^{102}R^{103}$, or $N=CR^{104}R^{105}$; wherein the meaning may have been chosen for each $R^{31}$ to $R^{38}$ independently of each other and wherein two or more of $R^{86}$ to $R^{93}$ may be linked to each other covalently;
wherein $R^{95}$, $R^{96}$, $R^{97}$, $R^{98}$, $R^{99}$, $R^{100}$, $R^{101}$, $R^{102}$, $R^{103}$ are selected from: hydrogen, substituted or unsubstituted, linear or branched, aliphatic or aromatic hydrocarbon group; $-OR^{104}$;
wherein $R^{104}$ is selected from: hydrogen, substituted or unsubstituted, linear or branched, aliphatic or aromatic hydrocarbon group; wherein $M^1$ and $M^2$ are selected from: alkali metal, alkaline earth metal, ammonium, phosphonium, and
wherein the meaning may have been chosen for $M^1$ and $M^2$ independently of each other.

**7.** Compound according to any of Claims 1 to 6,
wherein the $P^{III}(G^2)$ $(G^3)$ group corresponds in terms of structural formula to the $P^{III}(G^5)$ $(G^6)$ group.

**8.** Complex comprising:

- a compound according to any of Claims 1 to 7,
- at least one central metal atom,

wherein the compound is coordinated onto the central metal atom via at least one $P^{III}$.

**9.** Complex according to Claim 8,
wherein the central metal atom is selected from groups 8 to 10 of the periodic table.

**10.** Complex according to Claim 9, wherein the central metal atom is rhodium.

**11.** Composition containing:

- a compound according to any of Claims 1 to 7 which is not coordinated on a central metal atom, and
- a complex according to any of Claims 8 to 10.

**12.** Use of a composition according to Claim 11 as catalytically active composition in the synthesis of organic compounds.

**13.** Use of a composition according to Claim 11 as catalytically active composition in a process for hydroformylation of olefinically unsaturated hydrocarbon mixtures.

**14.** Multiphasic reaction mixture containing:

- an olefinically unsaturated hydrocarbon mixture,
- a gas mixture containing carbon monoxide and hydrogen,
- aldehydes,
- a composition according to Claim 11 as catalytically active composition.

**15.** Process for hydroformylation of olefinically unsaturated hydrocarbon mixtures to aldehydes comprising the steps of:

a) providing a mixture of olefinically unsaturated hydrocarbons;
b) adding a catalytically active composition according to Claim 11;
c) introducing a mixture comprising carbon monoxide and hydrogen;
d) heating the reaction mixture to a temperature in the range from 80 to 120°C;
e) building a pressure in the range from 1.0 to 6.4 MPa;
f) removing the olefinically unsaturated hydrocarbon mixture on concluding the reaction.

**16.** Process according to Claim 15,
comprising a further step:

g) removing and returning unconverted olefinically unsaturated hydrocarbon mixture into step a).

**17.** Process according to either Claim 15 or 16, comprising a further step:

h) removing and returning the catalytically active composition according to Claim 14 into step b) .

**18.** Process according to any of Claims 15 to 17, comprising a further step:

i) removing and returning the unconverted gas mixture containing carbon monoxide and hydrogen into step c) .

**Revendications**

**1.** Composé,
qui présente l'élément de structure (IV) :

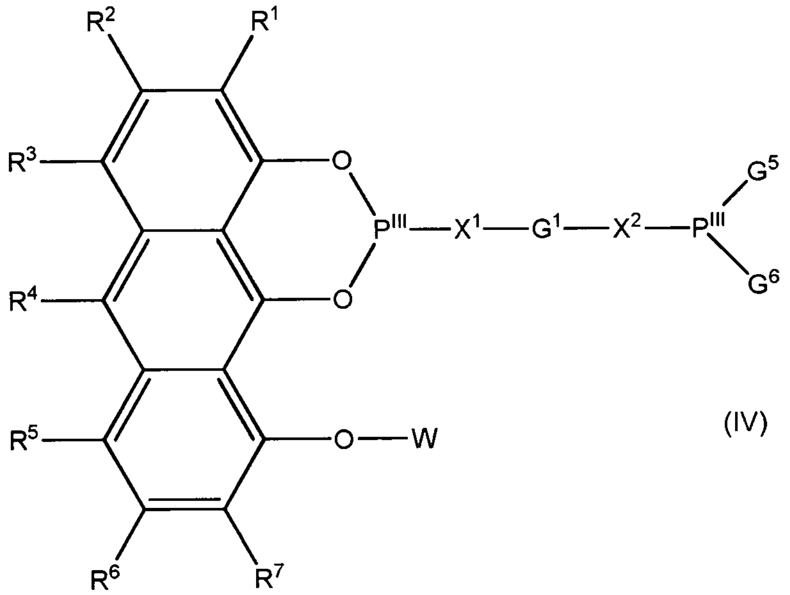

(IV)

$G^1$ = un groupe hydrocarboné linéaire ou ramifié, aliphatique ou aromatique ou hétéroaromatique ou aromatique condensé ou aromatique-hétéroaromatique, condensé, présentant une autre substitution quelconque ;
$X^1$, $X^2$ = O ;
$R^1$, $R^2$, $R^3$, $R^4$, $R^5$, $R^6$, $R^7$ étant choisis parmi : hydrogène, un groupe hydrocarboné non substitué ou substitué, linéaire ou ramifié, aliphatique ou aromatique ; F, Cl, Br, I, $-OR^8$, $-C(O)R^9$, $-CO_2R^{10}$, $-CO_2M^1$, $-SR^{11}$, $-SOR^{12}$, $-SO_2R^{13}$, $-SO_3R^{14}$, $-SO_3M^2$, $-NR^{15}R^{16}$ ;
$R^8$, $R^9$, $R^{10}$, $R^{11}$, $R^{12}$, $R^{13}$, $R^{14}$, $R^{15}$, $R^{16}$ étant choisis parmi : hydrogène, un groupe hydrocarboné non substitué ou substitué, linéaire ou ramifié, aliphatique ou aromatique ou hétéroaromatique ou aromatique condensé ou aromatique-hétéroaromatique condensé ; $-OR^{17}$ ;
$R^{17}$ étant choisi parmi : hydrogène, un groupe hydrocarboné non substitué ou substitué, linéaire ou ramifié, aliphatique ou aromatique ; deux des $R^1$ à $R^{17}$ ou plus pouvant être liés par covalence les uns aux autres ;
$M^1$ et $M^2$ étant choisis parmi : un métal alcalin, un métal alcalino-terreux, ammonium, phosphonium et les significations pour $M^1$ et $M^2$ pouvant être choisies indépendamment les unes des autres ;
W représentant un groupe $P^{III}(G^2)$ $(G^3)$ :

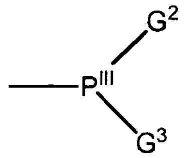

$G^2$ et $G^3$ étant chacun choisis parmi : hydrogène ; un groupe hydrocarboné linéaire ou ramifié, aliphatique ou aromatique ou hétéroaromatique ou aromatique condensé ou aromatique-hétéroaromatique condensé, présentant une autre substitution quelconque ; F, Cl, Br, I ou $-OR^{18}$, $-C(O)R^{19}$, $-CO_2R^{20}$, $-CO_2M^1$, $-SR^{21}$, $-SOR^{22}$, $-SO_2R^{23}$, $-SO_3R^{24}$,$-SO_3M^2$, $-NR^{25}R^{26}$, ;
$R^{18}$, $R^{19}$, $R^{20}$, $R^{21}$, $R^{22}$, $R^{23}$, $R^{24}$, $R^{25}$, $R^{26}$ étant choisis parmi : hydrogène, un groupe hydrocarboné non substitué ou substitué, linéaire ou ramifié, aliphatique ou aromatique ; $-OR^{27}$ ;
$R^{27}$ étant choisi parmi : hydrogène, un groupe hydrocarboné non substitué ou substitué, linéaire ou ramifié, aliphatique ou aromatique ; F, Cl, Br, I ;
les significations pour $G^2$ et $G^3$ pouvant être choisies indépendamment les unes des autres, et $G^2$ et $G^3$ étant liés par covalence l'un à l'autre,
$G^5$ et $G^6$ étant choisis parmi : hydrogène ; un groupe hydrocarboné linéaire ou ramifié, aliphatique ou aromatique ou hétéroaromatique ou aromatique condensé ou aromatique-hétéroaromatique condensé, présentant une autre substitution quelconque ; F, Cl, Br, I ou $-OR^{41}$, $-C(O)R^{42}$, $-CO_2R^{43}$, $-CO_2M^1$, $-SR^{44}$, $-SOR^{45}$, $-SO_2R^{46}$, $-SO_3R^{47}$,$-SO_3M^2$, $-NR^{48}R^{49}$,
$R^{41}$, $R^{42}$, $R^{43}$, $R^{44}$, $R^{45}$, $R^{46}$, $R^{47}$, $R^{48}$, $R^{49}$ étant choisis parmi : hydrogène, un groupe hydrocarboné non substitué ou substitué, linéaire ou ramifié, aliphatique ou aromatique ; $-OR^{50}$ ;

$R^{50}$ étant choisi parmi : hydrogène, un groupe hydrocarboné non substitué ou substitué, linéaire ou ramifié, aliphatique ou aromatique ;
$M^1$ et $M^2$ étant choisis parmi : un métal alcalin, un métal alcalino-terreux, ammonium, phosphonium et les significations pour $M^1$ et $M^2$ pouvant être les choisies indépendamment les unes des autres,
les significations pour $G^5$ et $G^6$ pouvant être choisies indépendamment les unes des autres et $G^5$ et $G^6$ pouvant être liés par covalence l'un à l'autre,
$G^1$ présentant l'élément de structure (V) :

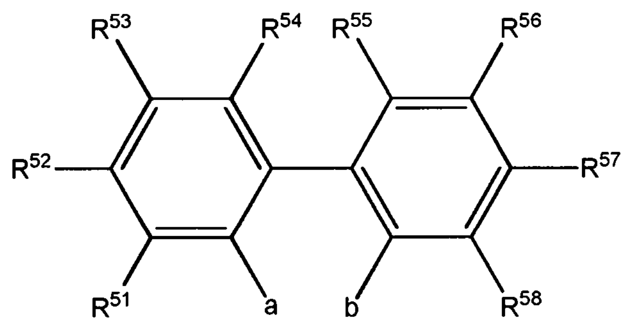

(V)

$R^{51}$, $R^{52}$, $R^{53}$, $R^{54}$, $R^{55}$, $R^{56}$, $R^{57}$, $R^{58}$ = hydrogène ; un groupe hydrocarboné linéaire ou ramifié, aliphatique ou aromatique ou hétéroaromatique ou aromatique condensé ou aromatique-hétéroaromatique condensé, présentant une autre substitution quelconque ; F, Cl, Br ou I ; ou $-OR^{59}$, $-COR^{60}$, $-CO_2R^{61}$, $-CO_2M^1$, $-SR^{62}$, $-SOR^{63}$, $-SO_2R^{64}$,$-SO_3R^{65}$, $-SO_3M^2$, $-NR^{66}R^{67}$ ou $N{=}CR^{68}R^{69}$ ; les significations pour $R^{51}$ à $R^{58}$ pouvant être choisies indépendamment les unes des autres et deux $R^{51}$ à $R^{58}$ ou plus pouvant être liés par covalence les uns aux autres ;
$R^{59}$, $R^{60}$, $R^{61}$, $R^{62}$, $R^{63}$, $R^{64}$, $R^{65}$, $R^{66}$, $R^{67}$ étant choisis parmi : hydrogène, un groupe hydrocarboné non substitué ou substitué, linéaire ou ramifié, aliphatique ou aromatique ; $-OR^{68}$ ;
$R^{68}$ étant choisi parmi : hydrogène, un groupe hydrocarboné non substitué ou substitué, linéaire ou ramifié, aliphatique ou aromatique ;
$M^1$ et $M^2$ étant choisis parmi : un métal alcalin, un métal alcalino-terreux, ammonium, phosphonium et les significations pour $M^1$ et $M^2$ pouvant être choisies indépendamment les unes des autres et
a et b étant les points de liaison à $X^1$ et $X^2$.

2. Composé selon la revendication 1, $G^2$, $G^3$ = $-OR^{18}$.

3. Composé selon l'une quelconque des revendications 1 ou 2, $G^5$, $G^6$ = $-OR^{41}$.

4. Composé selon l'une quelconque des revendications 1 à 3,
les liaisons $G^2$-$G^3$ présentant l'élément de structure suivant (VI) :

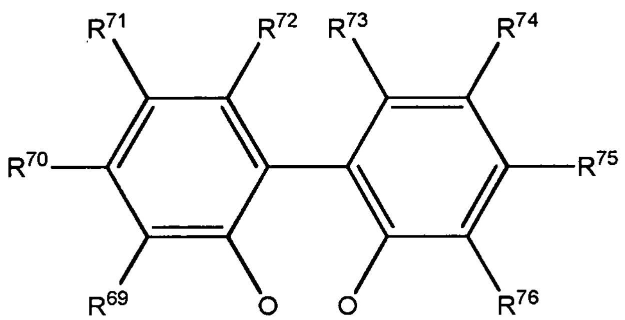

(VI)

$R^{69}$, $R^{70}$, $R^{71}$, $R^{72}$, $R^{73}$, $R^{74}$, $R^{75}$, $R^{76}$ = hydrogène ; un groupe hydrocarboné linéaire ou ramifié, aliphatique ou aromatique ou hétéroaromatique ou aromatique condensé ou aromatique-hétéroaromatique condensé présentant une autre substitution quelconque ; F, Cl, Br ou I ; ou $-OR^{77}$, $-COR^{78}$, $-CO_2R^{79}$, $-CO_2M^1$, $-SR^{80}$, $-SOR^{81}$, $-SO_2R^{82}$,$-SO_3R^{83}$, $-SO_3M^2$, $-NR^{84}R^{85}$ ou $N{=}CR^{86}R^{87}$ ; les significations pour $R^{69}$ à $R^{76}$ pouvant être choisies indépendamment les unes des autres et deux des $R^{69}$ à $R^{76}$ ou plus pouvant être liés par covalence les uns aux autres ;

$R^{77}$, $R^{78}$, $R^{79}$, $R^{80}$, $R^{81}$, $R^{82}$, $R^{83}$, $R^{84}$, $R^{85}$ étant choisi parmi : hydrogène, un groupe hydrocarboné non substitué ou substitué, linéaire ou ramifié, aliphatique ou aromatique ; $-OR^{86}$ ;
$R^{86}$ étant choisi parmi : hydrogène, un groupe hydrocarboné non substitué ou substitué, linéaire ou ramifié, aliphatique ou aromatique ;
$M^1$ et $M^2$ étant choisis parmi : un métal alcalin, un métal alcalino-terreux, ammonium, phosphonium, et les significations pour $M^1$ et $M^2$ pouvant être choisies indépendamment les unes des autres.

**5.** Composé selon l'une quelconque des revendications 1 à 4, $G^5$ et $G^6$ étant liés par covalence l'un à l'autre.

**6.** Composé selon l'une quelconque des revendications 1 à 5, les liaisons $G^5$-$G^6$ présentant l'élément de structure suivant (VII) :

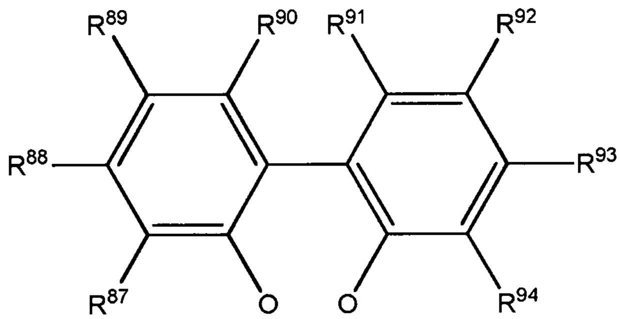

(VII)

$R^{87}$, $R^{88}$, $R^{89}$, $R^{90}$, $R^{91}$, $R^{92}$, $R^{93}$, $R^{94}$ = hydrogène ; un groupe hydrocarboné linéaire ou ramifié, aliphatique ou aromatique ou hétéroaromatique ou aromatique condensé ou aromatique-hétéroaromatique condensé, présentant une autre substitution quelconque ; F, Cl, Br ou I ; ou $-OR^{95}$, $-COR^{96}$, $-CO_2R^{97}$, $-CO_2M^1$, $-SR^{98}$, $-SOR^{99}$, $-SO_2R^{100}$,$-SO_3R^{101}$, $-SO_3M^2$, $-NR^{102}R^{103}$ ou $N=R^{104}R^{105}$ ; les significations pour $R^{31}$ à $R^{38}$ pouvant être choisies indépendamment les unes des autres et deux des $R^{86}$ à $R^{93}$ ou plus pouvant être liés par covalences les uns aux autres ;
$R^{95}$, $R^{96}$, $R^{97}$, $R^{98}$, $R^{99}$, $R^{100}$, $R^{101}$, $R^{102}$, $R^{103}$ pouvant être choisis parmi : hydrogène, un groupe hydrocarboné non substitué ou substitué, linéaire ou ramifié, aliphatique ou aromatique ; $-OR^{104}$ ;
$R^{104}$ étant choisi parmi : hydrogène, un groupe hydrocarboné non substitué ou substitué, linéaire ou ramifié, aliphatique ou aromatique ;
$M^1$ et $M^2$ étant choisis parmi : un métal alcalin, un métal alcalino-terreux, ammonium, phosphonium et les significations pour $M^1$ et $M^2$ pouvant être choisies indépendamment les unes des autres.

**7.** Composé selon l'une quelconque des revendications 1 à 6, le groupe $P^{III}(G^2)(G^3)$ correspondant à la formule de structure de $P^{III}(G^5)(G^6)$.

**8.** Composé complexe comprenant :

- un composé selon l'une quelconque des revendications 1 à 7,
- au moins un atome central métallique,

le composé étant coordiné via au moins un $P^{III}$ à l'atome central métallique.

**9.** Composé complexe selon la revendication 8, l'atome central métallique étant choisi dans les groupes 8 à 10 du système périodique des éléments.

**10.** Composé complexe selon la revendication 9, l'atome central métallique étant le rhodium.

**11.** Composition contenant

- un composé selon l'une quelconque des revendications 1 à 7, qui n'est pas coordiné un atome central métallique et
- un composé complexe selon l'une quelconque des revendications 8 à 10.

**12.** Utilisation d'une composition selon la revendication 11 comme composition catalytiquement active dans la synthèse

de composés organiques.

**13.** Utilisation d'une composition selon la revendication 11 comme composition catalytiquement active dans un procédé pour l'hydroformylation de mélanges hydrocarbonés oléfiniquement insaturés.

**14.** Mélange réactionnel à plusieurs phases, contenant :

- un mélange hydrocarboné oléfiniquement insaturé,
- un mélange gazeux contenant du monoxyde de carbone et de l'hydrogène,
- des aldéhydes,
- une composition selon la revendication 11 comme composition catalytiquement active.

**15.** Procédé pour l'hydroformylation de mélanges hydrocarbonés oléfiniquement insaturés en aldéhydes, comprenant les étapes de procédé, consistant à :

a) préparer un mélange d'hydrocarbures oléfiniquement insaturés ;
b) ajouter une composition catalytiquement active selon la revendication 11 ;
c) introduire un mélange qui présente du monoxyde de carbone et de l'hydrogène ;
d) chauffer le mélange réactionnel à une température dans la plage de 80 à 120°C ;
e) établir une pression dans la plage de 1,0 à 6,4 MPa ;
f) à la fin de la réaction, séparer le mélange hydrocarboné oléfiniquement insaturé.

**16.** Procédé selon la revendication 15, comprenant une autre étape de procédé, consistant à :

g) séparer et recycler le mélange hydrocarboné oléfiniquement insaturé non transformé dans l'étape de procédé a).

**17.** Procédé selon l'une quelconque des revendications 15 ou 16, comprenant une autre étape de procédé consistant à :

h) séparer et recycler la composition catalytiquement active selon la revendication 14 dans l'étape de procédé b).

**18.** Procédé selon l'une quelconque des revendications 15 à 17, comprenant une autre étape de procédé consistant à :

i) séparer et recycler le mélange gazeux non transformé, contenant du monoxyde de carbone et de l'hydrogène dans l'étape de procédé c).

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4694109 A
- US 4879416 A
- WO 9530680 A
- US 4169861 A
- US 4201714 A
- US 4193943 A
- EP 0155508 A
- EP 0214622 A
- EP 0472071 A
- US 4668651 A
- US 4748261 A
- US 4885401 A
- US 5312996 A
- EP 1294731 A
- EP 2280920 A


### In der Beschreibung aufgeführte Nicht-Patentliteratur


- **B. CORNILS ; W. A. HERRMANN.** Applied Homogeneous Catalysis with Organometallic Compounds. VCH, 1996, vol. 1 & 2